# FASCICULE DE BREVET EUROPEEN

(11) **EP 4 225 341 B1**
(45) Date de publication et mention de la délivrance du brevet: **16.09.2026**
(21) Numéro de dépôt: 21810064.2
(22) Date de dépôt: 08.10.2021
(51) Int. Cl.: A61K 35/747, A61K 39/40, A61P 31/04, A61P 11/00

(54) **COMBINAISON D'ANTICORPS INHALÉS AVEC DES AGENTS IMMUNOMODULATEURS POUR LE TRAITEMENT OU LA PRÉVENTION D'INFECTIONS RESPIRATOIRES**
KOMBINATION AUS INHALIERTEN ANTIKÖRPERN UND IMMUNMODULATORISCHEN MITTELN ZUR BEHANDLUNG ODER PRÄVENTION VON ATEMWEGSINFEKTIONEN
COMBINATION OF INHALED ANTIBODIES AND IMMUNOMODULATORY AGENTS FOR THE TREATMENT OR PREVENTION OF RESPIRATORY INFECTIONS

(30) Priorité: 08.10.2020 FR 2010296
(43) Date de publication de la demande: 16.08.2023
(73) Titulaire: Université de Tours, 37020 Tours Cedex 1 (FR); Institut National de la Recherche pour l'Agriculture, l'Alimentation et l'Environnement, 75007 Paris (FR); Institut National de la Santé et de la Recherche Médicale (INSERM), 75013 Paris (FR)
(72) Inventeur: HEUZE-VOURC'H, Nathalie, 37000 Tours (FR); SECHER, Thomas, 37230 Luynes (FR); THOMAS, Muriel, 91430 Igny (FR)
(74) Mandataire: Ipsilon
(86) Numéro de dépôt international: PCT/FR2021/051758
(87) Numéro de publication internationale: WO 2022/074348

(56) Documents cités:
- WO-A1-2010/091189
- WO-A1-2013/070615
- WO-A1-2015/196011
- WO-A2-02/064161
- CN-A- 103 097 412
- FR-A1- 2 917 623
- FR-A1- 3 094 378
- KR-A- 20120 050 480
- US-A1- 2012 263 715
- US-A1- 2014 271 653
- FAURE KARINE ET AL: "Effects of monoclonal anti-PcrV antibody on Pseudomonas aeruginosa-induced acute lung injury in a rat model", JOURNAL OF IMMUNE BASED THERAPIES AND VACCINES, BIOMED CENTRAL LTD., LONDON, GB, vol. 1, no. 1, 13 August 2003 (2003-08-13), pages 2, XP021008582, ISSN: 1476-8518, DOI: 10.1186/1476-8518-1-2
- AUDE REMOT ET AL: "Bacteria isolated from lung modulate asthma susceptibility in mice", THE I S M E JOURNAL: MULTIDISCIPLINARY JOURNAL OF MICROBIAL ECOLOGY, vol. 11, no. 5, 3 January 2017 (2017-01-03), United Kingdom, pages 1061 - 1074, XP055641706, ISSN: 1751-7362, DOI: 10.1038/ismej.2016.181
- AUDE REMOT ET AL: "Bacteria Isolated From Lung Modulate Asthma Susceptibility In Mice; Supplementary Table S2: Descriptive table of the 20 isolated mouse lung strains", 3 January 2017 (2017-01-03), XP055642205, Retrieved from the Internet <URL:https://static-content.springer.com/esm/art%3A10.1038%2Fismej.2016.181/MediaObjects/41396_2017_BFismej2016181_MOESM6_ESM.pdf> [retrieved on 20191113]
- JULIO VILLENA ET AL: "Lactic acid bacteria in the prevention of pneumococcal respiratory infection: Future opportunities and challenges", INTERNATIONAL HNMUNOPHARMACOLOGY, ELSEVIER, AMSTERDAM, NL, vol. 11, no. 11, 7 June 2011 (2011-06-07), pages 1633 - 1645, XP028326740, ISSN: 1567-5769, [retrieved on 20110617], DOI: 10.1016/J.INTIMP.2011.06.004
- BERNARD-RAICHON LUCIE ET AL: "A Pulmonary Lactobacillus murinus Strain Induces Th17 and ROR[gamma]t + Regulatory T Cells and Reduces Lung Inflammation in Tuberculosis", THE JOURNAL OF IMMUNOLOGY, vol. 207, no. 7, 3 September 2021 (2021-09-03), US, pages 1857 - 1870, XP055882625, ISSN: 0022-1767, DOI: 10.4049/jimmunol.2001044
- BERNARD LUCIE: "Use of lung microbiota strains to shape local immunity at steady state and during Mycobacterium tuberculosis infection in the mouse model", 13 January 2020 (2020-01-13), XP055882634, Retrieved from the Internet <URL:chrome-extension://efaidnbmnnnibpcajpcglclefindmkaj/viewer.html?pdfurl=https%3A%2F%2Ftel.archives-ouvertes.fr%2Ftel-03169776%2Fdocument&clen=10841413> [retrieved on 20220124]
- SÉCHER THOMAS ET AL: "In a murine model of acute lung infection, airway administration of a therapeutic antibody confers greater protection than parenteral administration", JOURNAL OF CONTROLLED RELEASE, vol. 303, 10 June 2019 (2019-06-10), pages 24 - 33, XP085712820, ISSN: 0168-3659, DOI: 10.1016/J.JCONREL.2019.04.005

## Description

### DOMAINE TECHNIQUE

La présente invention concerne le traitement et la prévention d'infections respiratoires chez un sujet. En particulier, la présente invention concerne le traitement ou la prévention d'infections respiratoires causées par des bactéries, par exemple, des bactéries résistantes aux antibiotiques, et plus particulièrement, *Pseudomonas aeruginosa.*

### ÉTAT DE LA TECHNIQUE

Les maladies respiratoires constituent un enjeu majeur en santé publique à l'échelle mondiale, avec 4 familles de pathologies respiratoires figurant parmi les 10 premières causes de mortalité et totalisant près de 9 millions de décès par an (soit 1/6 des décès totaux), auxquels s'ajoutent une morbidité significative et un fort impact économique (380 Mds€ annuels en Europe). Ces maladies incluent notamment la broncho-pneumopathie chronique obstructive (BPCO), le cancer du poumon, les infections respiratoires aigües et la tuberculose.

En particulier, les infections respiratoires représentent un quart des décès dus aux maladies respiratoires en Europe. Au niveau mondial, les infections respiratoires (en excluant la tuberculose) ont causé 2,4 millions de décès en 2016, les positionnant au 6^{ème} rang des maladies les plus meurtrières chez l'homme (tous âges confondus), et comme la première cause de mortalité chez les enfants de moins de 5 ans. La prévention et le traitement des infections respiratoires représentent donc un enjeu majeur de santé publique.

Différentes approches ont été proposées pour le traitement des infections respiratoires.

La demande WO 2010/091189 A1 décrit l'utilisation combinée d'un anticorps anti-PcrV et d'un antibiotique pour le traitement d'une infection pulmonaire par *Pseudomonas aeruginosa.*

La demande WO 2013/070615 A1 décrit un anticorps se liant à PcrV de *Pseudomonas* pour prévenir ou traiter une infection respiratoire, l'administration de l'anticorps par inhalation étant décrite, en combinaison avec un antibiotique.

La demande WO 2015/196011 A1 décrit l'administration combinée d'un anticorps se liant à un antigène de *P. aeruginosa* et d'un agent antibactérien, l'administration de l'anticorps par inhalation étant décrite.

La demande WO 02/064161 A2 décrit une méthode de traitement d'une infection respiratoire par *P. aeruginosa* au moyen de l'anticorps Mab166.

La publication scientifique de Faure et al. (Journal of Immune Based Therapies and Vaccines, 2003, vol. 1, no. 1, page 2) décrit l'utilisation de mAb166 dans le traitement d'affections respiratoires causées par *P. aeruginosa.*

La demande FR 3 094 378 A1 décrit l'utilisation de bactéries du microbiote pulmonaire, notamment des lactobacilles, pour le traitement de l'inflammation liée à une maladie respiratoire.

La demande FR 2 917 623 A1 décrit l'utilisation de bactéries *Lactobacillus* pour prévenir la colonisation du système respiratoire par des bactéries pathogènes.

La publication scientifique de Remot et al. (The ISME Journal, 2017, vol. 11, no. 5, pages 1061-1074) décrit l'isolation de souches Lactobacillaceae de poumons de souris et suggère l'utilisation de probiotiques pour l'immunité pulmonaire.

La publication scientifique de Villena et al. (International Immunopharmacology, 2011, vol. 11, no. 11, pages 1633-1645) décrit l'utilisation de bactéries lactiques dans le traitement d'infections respiratoires et leur effet immunomodulateur.

La publication scientifique de Bernard-Raichon et al. (The Journal of Immunology, 2021, vol. 207, no. 7, pages 1857-1870) décrit l'utilisation de la souche *Lactobacillus murinus* pour réduire l'inflammation pulmonaire.

La publication scientifique de Secher et al. (Journal of Controlled Release, 2019, vol. 303, pages 24-33) montre que l'administration d'un anticorps directement dans les voies aériennes confère plus de protection qu'une administration parentérale dans le traitement de la pneumonie.

La demande US 2012/263715 A1 décrit une méthode de réduction de la charge virale du virus respiratoire syncytial comprenant l'administration d'un anticorps anti-RSV par inhalation.

Les demandes US 2014/271653 A1, CN 103 097 412 A et KR20120050480 décrivent des compositions pharmaceutiques comprenant un anticorps anti-VRS formulées pour une administration pulmonaire ou intranasale.

Bien que les antibiotiques aient révolutionné la prise en charge de ces pathologies, leur efficacité décroît continuellement du fait de l'émergence rapide de phénomènes de résistance. Au vu du faible nombre de nouveaux antibiotiques commercialisés, les agences gouvernementales réclament que des efforts soient faits pour développer de nouvelles stratégies thérapeutiques anti-infectieuses.

Ainsi, les Inventeurs ont développé une stratégie innovante basée sur une combinaison d'anticorps administrés par inhalation et d'agents immunomodulateurs pour lutter contre les infections respiratoires. Les Inventeurs ont notamment montré que l'administration pulmonaire d'un anticorps dirigé contre un agent infectieux, en combinaison avec des souches probiotiques, permet de traiter l'infection respiratoire causée par l'agent infectieux et de prévenir sa réapparition.

### RÉSUMÉ DE L'INVENTION

La présente invention a pour objet une combinaison d'au moins un agent capable de lier un agent infectieux respiratoire, ledit agent étant choisi parmi anticorps, un anticorps bispécifique, un anticorps multispécifique, un fragment d'anticorps, un anticorps à domaine unique, un unibody, un nanobody, un affibody, une affiline, une affitine, une adnectine, une atrimère, une DARPin, une anticaline, une avimère, une fynomère ou un versabody, et d'au moins un agent immunostimulant, pour son utilisation dans le traitement ou la prévention à long-terme d'une infection respiratoire et d'une réinfection respiratoire chez un sujet, dans laquelle l'au moins un agent capable de lier l'agent infectieux respiratoire est à administrer par inhalation chez le sujet, étant entendu par long-terme que le traitement ou la prévention perdure alors que l'au moins un agent capable de lier l'agent infectieux n'est plus détecté dans l'organisme et/ou lorsqu'une réponse immunitaire adaptative est induite dans l'organisme.

Selon un mode de réalisation, l'au moins un agent capable de lier l'agent infectieux respiratoire est pour administration par inhalation chez le sujet. Selon un mode de réalisation, l'au moins un agent capable de lier l'agent infectieux respiratoire est sous une forme adaptée à une administration par inhalation chez le sujet.

Comme indiqué ci-dessus, la sélection dudit au moins un agent capable de lier l'agent infectieux respiratoire est faite au sein d'une liste comprenant des anticorps, des dérivés d'anticorps et des mimétiques d'anticorps.

Selon un mode de réalisation, l'au moins un agent immunostimulant est sélectionné parmi le groupe comprenant ou consistant en une souche probiotique, un mélange de souches probiotiques, un agoniste des récepteurs de type Toll, un agoniste des récepteurs de type NOD, un agonistes des récepteurs de type RIG, une cytokine ou un mélange de cytokines, une chémokine ou un mélange de chémokines, un adjuvant tel que le chitosan, une flagelline, un variant de flagelline dans lequel un ou plusieurs résidu(s) d'acide aminé donné a/ont été modifié(s) sans altérer la conformation globale et la fonction de la flagelline, un polypeptide comprenant ou consistant en un ou plusieurs fragment(s) de flagelline, un oligodésoxynucléotide CpG (CpG ODN), l'α-galactosylcéramide (α-Gal-Cer), les sels d'aluminium (hydroxyde d'aluminium, phosphate d'aluminium, et sulfate de potassium et d'aluminium), le MF59, le AS03, l'acide polyinosinique-polycytidylique, un polyphosphazène, un anticorps dirigé contre les points du contrôle du système immunitaire tels que CTLA-4, PD-1, PD-L1 ou CD137 et leurs mélanges.

Selon un mode de réalisation, l'au moins un agent immunostimulant est ou comprend une souche probiotique ou un mélange de souches probiotiques sélectionnée(s) parmi la famille *Lactobacillaceae,* préférentiellement parmi l'espèce *Lactobacillus murinus.*

Selon un mode de réalisation, l'au moins un agent immunostimulant est ou comprend une souche probiotique sélectionnée parmi les souches déposées le 14 avril 2015 à la Collection Nationale de Cultures de Microorganismes (CNCM) sous les numéros CNCM I-4967 et CNCM I-4968, ou la souche déposée à la CNCM le 16 Avril 2018 sous le numéro CNCM I-5314, ou un mélange de celles-ci.

Selon un mode de réalisation, l'au moins un agent immunostimulant est ou comprend un mélange des souches probiotiques CNCM I-4967 et CNCM I-4968. Selon un mode de réalisation, l'au moins un agent immunostimulant est ou comprend un mélange des souches probiotiques CNCM I-4967 et CNCM I-5314. Selon un mode de réalisation, l'au moins un agent immunostimulant est ou comprend un mélange des souches probiotiques CNCM I-5314 et CNCM I-4968. Selon un mode de réalisation, l'au moins un agent immunostimulant est ou comprend un mélange des souches probiotiques CNCM I-4967, CNCM I-4968 et CNCM I-5314.

Selon un mode de réalisation, l'agent infectieux respiratoire est sélectionné parmi le groupe comprenant ou consistant en les virus, bactéries, champignons et parasites, préférentiellement l'agent infectieux respiratoire est au moins temporairement extracellulaire.

Selon un mode de réalisation, l'agent infectieux respiratoire est une bactérie, préférentiellement une bactérie résistante à un ou plusieurs antibiotiques, plus préférentiellement une bactérie sélectionnée parmi le groupe ESKAPE, encore plus préférentiellement une bactérie sélectionnée dans le groupe comprenant ou consistant en *Pseudomonas aeruginosa, Klebsiella pneumoniae, Staphylococcus aureus* et *Acinetobacter baumanii,* encore plus préférentiellement *Pseudomonas aeruginosa.*

Selon un mode de réalisation, l'au moins un agent capable de lier l'agent infectieux respiratoire est dirigé contre une molécule présente à la surface d'une bactérie. Selon un mode de réalisation, l'au moins un agent capable de lier l'agent infectieux respiratoire est dirigé contre une molécule présente à la surface de *Pseudomonas aeruginosa,* préférentiellement l'au moins un agent capable de lier l'agent infectieux respiratoire est dirigé contre une protéine du système de sécrétion de type III de *Pseudomonas aeruginosa.*

Selon un mode de réalisation, ladite infection respiratoire est une infection respiratoire aigüe, préférentiellement une infection respiratoire aigüe des voies inférieures, plus préférentiellement la bronchite, la bronchiolite, la pneumonie (incluant la pneumopathie nosocomiale, la pneumopathie communautaire, ou la pneumopathie acquise sous ventilation mécanique), la grippe ou la coqueluche.

Selon un mode de réalisation, le sujet souffre d'une pathologie respiratoire chronique, préférentiellement une pathologie respiratoire chronique sélectionnée parmi le groupe comprenant ou consistant en la bronchopneumopathie chronique obstructive (BPCO), les maladies interstitielles pulmonaires, le cancer du poumon, l'asthme (de l'adulte et de l'enfant), la bronchiectasie, les maladies rares et orphelines du poumon comme la mucoviscidose et les maladies vasculaires pulmonaires.

L'invention concerne également une composition comprenant ou consistant essentiellement en une combinaison d'au moins un agent capable de lier un agent infectieux respiratoire et d'au moins un agent immunostimulant pour son utilisation telle que décrite précédemment.

L'invention concerne également un nécessaire de pièces comprenant au moins deux parties, la première partie comprenant au moins un agent capable de lier l'agent infectieux respiratoire, ledit agent étant sélectionné parmi un anticorps, un anticorps bispécifique, un anticorps multispécifique, un fragment d'anticorps, un anticorps à domaine unique, un unibody, un nanobody, un affibody, une affiline, une affitine, une adnectine, une atrimère, une DARPin, une anticaline, une avimère, une fynomère et un versabody, et la seconde partie comprenant au moins un agent immunostimulant pour son utilisation dans le traitement ou la prévention à long-terme d'une infection respiratoire et d'une réinfection respiratoire chez un sujet, dans lequel l'au moins un agent capable de lier l'agent infectieux respiratoire est à administrer par inhalation chez le sujet, étant entendu par long-terme que le traitement ou la prévention perdure alors que l'au moins un agent capable de lier l'agent infectieux n'est plus détecté dans l'organisme et/ou lorsqu'une réponse immunitaire adaptative est induite dans l'organisme.

Comme indiqué ci-dessus, la sélection dudit au moins un agent capable de lier l'agent infectieux respiratoire est faite au sein d'une liste comprenant des anticorps, des dérivés d'anticorps et des mimétiques d'anticorps.

Selon un mode de réalisation, l'au moins un agent immunostimulant est sélectionné parmi le groupe comprenant ou consistant en une souche probiotique, un mélange de souches probiotiques, un agoniste des récepteurs de type Toll, un agoniste des récepteurs de type NOD, un agonistes des récepteurs de type RIG, une cytokine ou un mélange de cytokines, une chémokine ou un mélange de chémokines, un adjuvant tel que le chitosan, une flagelline, un variant de flagelline dans lequel un ou plusieurs résidu(s) d'acide aminé donné a/ont été modifié(s) sans altérer la conformation globale et la fonction de la flagelline, un polypeptide comprenant ou consistant en un ou plusieurs fragment(s) de flagelline, un oligodésoxynucléotide CpG (CpG ODN), l'α-galactosylcéramide (α-Gal-Cer), les sels d'aluminium (hydroxyde d'aluminium, phosphate d'aluminium, et sulfate de potassium et d'aluminium), le MF59, le AS03, l'acide polyinosinique-polycytidylique, un polyphosphazène, un anticorps dirigé contre les points du contrôle du système immunitaire tels que CTLA-4, PD-1, PD-L1 ou CD137 et leurs mélanges.

### DÉFINITIONS

Dans la présente invention, les termes ci-dessous sont définis de la manière suivante :
- "Adnectine" : désigne une protéine artificielle se liant à un antigène, basée sur le 10^{ème} domaine extracellulaire de la fibronectine type III.
- "Affibody" : désigne une protéine d'affinité basée sur un domaine protéique de 58 acides aminés dérivé d'un domaine de liaison à l'immunoglobuline G de la protéine A de la bactérie *Staphylococcus aureus.*
- "Affiline" : désigne une protéine artificielle se liant à un antigène, et dont la structure dérive de la gamma-B-crystalline humaine.
- "Affitine" : désigne une protéine se liant à un antigène, dont la structure dérive de la protéine Sac7d, se liant à l'ADN, issue de *Sulfolobus acidocaldarius.* Des exemples d'affitines incluent, sans y être limités, les Nanofitines développées par Affilogic. Selon un mode de réalisation, l'au moins un agent capable de lier un agent infectieux est une affitine, par exemple, une Nanofitine.
- "Atrimère" : désigne une protéine artificielle se liant à un antigène, basée sur la structure d'une molécule humaine trivalente, la tétranectine.
- "Agent immunomodulateur" : désigne une substance caractérisée par sa capacité à moduler directement le système immunitaire, soit en le stimulant (l'agent immunomodulateur pouvant alors être désigné par le terme «agent immunostimulant ») ou le freinant. Selon un mode de réalisation, la substance est ajoutée pour assister ou coopérer avec le système immunitaire pour augmenter les réactions immunitaires contre l'agent infectieux. Dans un mode de réalisation, l'agent immunomodulateur peut donc être désigné par le terme « agent immunostimulant ».
- "Agent infectieux" : désigne un agent responsable d'une maladie infectieuse respiratoire. Les agents infectieux incluent notamment les virus, bactéries, champignons et parasites. Au sens de la présente invention, le terme « agent infectieux » désigne notamment les agents infectieux (ou agents pathogènes) émergents.
- "Agoniste" : désigne une molécule qui se lie à un récepteur et qui l'active pour induire une réponse biologique.
- "Antibiotique" : désigne une substance naturelle ou synthétique qui détruit ou inhibe la croissance des bactéries. Au sens de la présente invention, un agent antibiotique n'est pas considéré comme un agent immunomodulateur. Selon un mode de réalisation, un agent antibiotique n'est pas considéré comme un agent immunostimulant.
- "Anticaline" : désigne un mimétique d'anticorps pour lequel la spécificité de liaison est dérivée des lipocalines. Les anticalines peuvent être aussi être produites sous la forme de protéines se liant à 2 cibles, on parle alors de duocalines.
- "Anticorps" (qui peut également être désigné "Immunoglobuline") : désigne une molécule se liant à un antigène de façon spécifique. Un anticorps est généralement constitué de deux chaînes légères (L) et de deux chaînes lourdes (H) reliées entre elles par des liaisons covalentes ou non . Le terme générique « Immunoglobuline » (Ig) comprend 5 classes distinctes d'anticorps qui peuvent être distinguées biochimiquement. Au sens de la présente invention, les 5 classes d'anticorps sont couvertes par l'invention ; la discussion suivante sera principalement axée sur la classe G des immunoglobulines. Les immunoglobulines G comprennent deux chaînes polypeptidiques légères d'un poids moléculaire d'environ 23 000 Daltons (Da), et deux chaînes polypeptidiques lourdes d'un poids moléculaire d'environ 53 000-70 000 Da. Les 4 chaînes sont reliées par des ponts disulfures dans une configuration en forme de Y. Les chaînes légères d'un anticorps sont classées comme kappa ([κ]) ou lambda ([λ]). Chaque classe de chaîne lourde peut se lier à une chaîne légère [κ] ou [λ]. En général, les chaînes lourdes et légères sont liées de façon covalente les unes aux autres, et les régions de la queue des 2 chaînes lourdes sont liées entre elles par des liaisons disulfures covalentes ou des liaisons non covalentes lorsque les immunoglobulines sont produites par des hybridomes, des cellules B ou des cellules-hôtes génétiquement modifiées. Dans la chaîne lourde, la séquence d'acides aminés s'étend de la partie N terminale au niveau des branches du Y jusqu'à la partie C terminale à la fin de la chaîne. Les chaînes lourdes sont classées comme gamma, mu, alpha, delta, ou epsilon (γ, µ, α, δ, ε respectivement) avec, parmi elles, des sous-classes (*e.g*., γ1 - γ4). La nature de cette chaîne lourde détermine la classe (ou l'isotype) de l'anticorps, à savoir IgG, IgM, IgA IgD, ou IgE, respectivement. Des versions modifiées de chacune de ces classes et isotypes sont à la portée de l'homme du métier et sont donc comprises dans la présente invention. La partie variable d'un anticorps permet à l'anticorps de reconnaître de façon sélective des antigènes et de lier de façon spécifique l'épitope aux antigènes. En effet, le domaine variable de la chaîne légère et le domaine variable de la chaîne lourde combinés forment une région variable qui définit un domaine de liaison à l'antigène en 3 dimensions. Ce domaine est présent au bout de chaque branche du Y. De façon plus spécifique, le domaine de liaison à l'antigène est défini par 3 régions déterminant la complémentarité (complementarity determining regions, CDR) présentes sur chacune des chaînes lourdes et légères.
- "Anticorps à domaine unique" (Single domain antibody) : désigne la plus petite unité fonctionnelle de liaison à l'antigène d'un anticorps, qui correspond aux régions variables des chaines lourdes ou légères des anticorps.
- "Anticorps bispécifique" et "Anticorps multispécifique" : désigne un type d'anticorps modifié pour pouvoir se lier à deux (bispécifique) ou plusieurs (multispécifique) antigènes distincts.
- "Anticorps chimérique" : désigne un anticorps dans lequel les 2 types de chaînes (lourdes et légères) sont chimériques à la suite de modifications par ingénierie. Une chaîne chimérique est une chaîne contenant une région variable étrangère, *i.e.* issue d'une espèce autre que l'homme ou synthétique, liée à une région constante d'origine humaine.
- "Anticorps humanisé" : désigne un anticorps dans lequel les 2 types de chaînes sont humanisées à la suite de modifications par ingénierie. Une chaîne humanisée est une chaine dans laquelle les régions déterminant la complémentarité (CDR) des régions variables sont étrangères, *i.e.* issues d'une espèce autre que l'homme ou synthétique, alors que le reste de la chaîne est d'origine humaine.
- "Anticorps d'origine humaine" : désigne un anticorps dans lequel les 2 chaînes sont d'origine humaine.
- "Avimère" : désigne une protéine artificielle se liant à un antigène, dérivée de domaines de récepteurs membranaires.
- "Bactérie" : désigne un organisme procaryote, c'est-à-dire un organisme unicellulaire et sans noyau, dont le génome est constitué d'ADN. Celui-ci consiste en un seul chromosome, qui peut être accompagné de plasmides (ADN circulaire). On distingue deux catégories générales de bactéries en fonction des caractéristiques biochimiques de leur paroi cellulaire : les bactéries à Gram positif et les bactéries à Gram négatif.
- "Champignon" : désigne un organisme eucaryote hétérotrophe assurant sa nutrition par absorption et produisant des spores. On distingue deux groupes principaux : les ascomycètes et les basidiomycètes.
- "Chitosan" : désigne un polysaccharide linéaire composé de la distribution aléatoire de D-glucosamine liée en β-(1-4) et de N-acétyl-D-glucosamine.
- "DARPin" (Designed Ankyrin Repeat Proteins) : désigne un mimétique d'anticorps produit génétiquement et dérivé d'une protéine ankyrine.
- « Dérivé d'anticorps » désigne une molécule dérivée d'un anticorps tel que défini ci-dessus, et inclut notamment les anticorps bispécifiques et multispécifiques, les fragments d'anticorps, les anticorps à domaine unique (single domain antibodies), les unibodies et nanobodies.
- "Diabody" : désigne un dimère de fragments d'anticorps, chaque polypeptide étant constitué d'une région variable d'une chaîne lourde et d'une région variable d'une chaîne légère. Les triabodies et tétrabodies sont des trimères et tétramères de fragments respectivement.
- "Environ" : précédent un chiffre ou un nombre, désigne plus ou moins 10% de la valeur dudit chiffre ou dudit nombre.
- "Excipient pharmaceutiquement acceptable" : désigne une substance qui ne produit pas de réaction défavorable, allergique ou indésirable lorsqu'elle est administrée à un sujet. Cela inclut tous les solvants, les milieux de dispersion, les enrobages, les agents antibactériens et antifongiques, les agents isotoniques, les agents à absorption retardée et autres substances similaires. Pour une administration chez un être humain, les préparations doivent répondre aux critères de stérilité, de pyrogénicité, et aux normes de sécurité et de pureté générales requises par les offices régulateurs tels que la FDA ou l'EMA.
- « Flagelline » : désigne une protéine contenue dans une variété d'espèces bactériennes Gram positives ou Gram négatives. Des sources de flagellines, incluent, de façon non limitative, *Escherichia,* (*e.g., E. coli), Enterobacter, Erwinia, Klebsiella, Proteus, Salmonella (e.g., Salmonella enterica serovar Typhimurium), Serratia (e.g., Serratia marcescans), Shigella, Bacilli* (*e.g., B. subtilis* et *B. licheniformis), Pseudomonas* (*e.g., P. aeruginosa),* et *Streptomyces.* Les séquences d'acides aminés et les séquences nucléotidiques des flagellines sont disponibles publiquement dans la base de données « NCBI Genbank », par exemple sous les numéros d'accessions suivants : AAL20871, NP_310689, BAB58984, AAO85383, AAA27090, NP_461698, AAK58560, YP_001217666, YP_002151351, YP_001250079, AAA99807, CAL35450, AAN74969, et BAC44986. Les séquences de flagellines issues de ces espèces mais aussi d'autres espèces sont compris dans le terme « flagelline » utilisé ici. Ainsi, les différences de séquences entre espèces sont comprises dans ce terme.
- "Fragment d'anticorps" : désigne une partie ou une région d'un anticorps comprenant moins d'acides aminés que l'anticorps intact et pouvant se lier au même antigène et/ou pouvant être en compétition avec l'anticorps dont il dérive pour la liaison à l'antigène. Les fragments d'anticorps inclus de façon non limitative dans la présente invention sont les fragments Fab, Fab', F(ab')₂, Fd, les fragments variables (Fv), les fragments variables à chaine unique (scFv), les diabodies, triabodies et tétrabodies.
- "Fragment Fab" : désigne un fragment d'anticorps formé de la totalité de la chaîne légère (domaine variable de la chaîne légère (VL) et domaine constant de la chaîne légère (CL)) et d'une partie de la chaîne lourde (domaine variable de la chaîne lourde (VH) et domaine constant 1 de la chaîne lourde (CH1)).
- "Fragment Fab'" : désigne un fragment d'anticorps formé par la réduction d'un fragment F(ab')₂. Ce fragment contient notamment des groupes réactifs sulfhydryles.
- "Fragment F(ab')₂" : désigne l'association de deux fragments Fab reliés par une petite partie des parties constantes des chaînes lourdes, la région charnière.
- "Fragment Fc" : désigne un fragment d'anticorps constitué des domaines constants 2 et 3 des chaînes lourdes (CH2 et CH3) au-delà de la région charnière.
- "Fragment Fd" : désigne un fragment d'anticorps constitué de la région variable de la chaîne lourde (VH) et du premier domaine constant de la chaîne lourde (CH1).
- "Fragment variable (Fv)" : désigne un fragment d'anticorps constitué uniquement des régions variables des chaînes lourdes et légères (VL et VH).
- "Fragment variable à chaine unique (scFv)" : désigne les fragments d'anticorps comprenant les régions variables des chaines lourdes et légères (VH et VL) connectés en une seule chaîne d'acides aminés. Selon un mode de réalisation, un fragment scFv comprend un peptide linker entre les régions variables VH et VL permettant au fragment d'avoir la structure adéquate pour lier l'antigène (Plückthun, 1994. Antibodies from Escherichia coli. In Rosenberg & Moore (Eds.), The pharmacology of monoclonal antibodies. Handbook of Experimental Pharmacology, 113:269-315. Springer: Berlin, Heidelberg).
- "Fynomère" : désigne une protéine artificielle se liant à un antigène, dérivée du domaine SH3 de la protéine Fyn.
- "Identité" ou "identique" : lorsqu'il est utilisé dans une relation entre les séquences de deux polypeptides ou plus, désigne le degré de parenté de séquence entre les polypeptides, tel que déterminé par le nombre de correspondances entre les chaînes de deux résidus d'acides aminés ou plus. L'identité mesure le pourcentage de correspondances identiques entre les plus petites séquences de deux ou plusieurs séquences avec des alignements à trous (le cas échéant) traités par un modèle mathématique ou un programme informatique particulier (c'est-à-dire des "algorithmes"). L'identité de polypeptides apparentés peut être facilement calculée par des méthodes connues de l'homme du métier. Ces méthodes comprennent, sans s'y limiter, celles décrites dans Computational Molecular Biology, Lesk, A. M., ed., Oxford University Press, New York, 1988 ; Biocomputing : Informatics and Genome Projects, Smith, D. W., ed., Academic Press, New York, 1993 ; Computer Analysis of Sequence Data, Part 1, Griffin, A. M., and Griffin, H. G., eds, Humana Press, New Jersey, 1994 ; Sequence Analysis in Molecular Biology, von Heinje, G., Academic Press, 1987 ; Sequence Analysis Primer, Gribskov, M. and Devereux, J., eds., M. Stockton Press, New York, 1991 ; et Carillo et al., SIAM J. Applied Math. 48, 1073 (1988). Les méthodes préférées pour déterminer l'identité sont conçues pour donner la plus grande correspondance entre les séquences testées. Les méthodes de détermination de l'identité sont décrites dans des programmes informatiques accessibles au public. Les méthodes préférées de programmes informatiques pour déterminer l'identité entre deux séquences comprennent le progiciel GCG, y compris GAP (Devereux et coll. Acid. Res. \2, 387 (1984); Genetics Computer Group, University of Wisconsin, Madison, Wis.), BLASTP, BLASTN, et FASTA (Altschul et al., J. MoI. Biol. 215, 403-410 (1990)). Le programme BLASTX est disponible publiquement auprès du National Center for Biotechnology Information (NCBI) et d'autres sources (BLAST Manual, Altschul et al. NCB/NLM/NIH Bethesda, Md. 20894 ; Altschul et al., supra). L'algorithme bien connu de Smith Waterman peut également être utilisé pour déterminer l'identité.
- "Infection respiratoire" : désigne une atteinte de l'une des structures composant le système respiratoire, *i.e.* le nez, la gorge, le larynx, la trachée, les bronches ou les poumons par un agent infectieux.
- "Inhalation" désigne l'administration d'une substance dans le tractus respiratoire, notamment sous la forme d'un aérosol, d'un spray, de jets, de gouttes à gouttes ou de toute autre forme adaptée à l'administration directement dans le tractus respiratoire.
- "Mimétique d'anticorps" : désigne des molécules créées artificiellement et pouvant se lier de façon spécifique à des antigènes, comme le font les anticorps, mais ne partageant pas la structures des anticorps. Selon un mode de réalisation, les mimétiques d'anticorps sont couplés à un fragment Fc d'anticorps. Les mimétiques d'anticorps de la présente demande incluent de façon non limitative les affibodies, les affilines, les affitines, les adnectines, les atrimères, les DARPins, les anticalines, les avimères, les fynomères et les versabodies.
- "Morbidité" : désigne l'ensemble des effets subséquents à une maladie ou un traumatisme, souvent qualifiés de séquelles.
- "Nanobody" : désigne une protéine thérapeutique dérivée d'un anticorps contenant les propriétés structurelles et fonctionnelles d'anticorps à chaîne lourde présents dans la nature. Ces anticorps à chaîne lourde peuvent contenir un seul domaine variable et 2 domaines constants (CH2 et CH3).
- "Quantité thérapeutiquement efficace" : désigne la quantité nécessaire et suffisante de la combinaison de la présente invention à administrer chez un sujet permettant la prévention de l'infection respiratoire, le ralentissement ou l'arrêt de la progression, l'aggravation, la détérioration d'au moins un des symptômes de l'infection respiratoire. Cette quantité administrée peut permettre le soulagement des symptômes de l'infection respiratoire, la guérison de l'infection ou la prévention de l'infection respiratoire.
- "Parasite" désigne un organisme vivant sur ou dans un autre organisme, appelé hôte, aux dépens de celui-ci.
- "Peptide" désigne un polymère linéaire d'acides aminés, généralement composé de moins de 50 acides aminés liés entre eux par des liaisons peptidiques. Les polymères d'acides aminés composés de plus de 50 acides aminés sont généralement appelés polypeptides, tandis que le terme "Protéine" désigne généralement un assemblage de peptides ou polypeptides.
- « Polypeptide comprenant ou consistant en un ou plusieurs fragment(s) de flagelline » ou « Polypeptide de flagelline » : désigne un polypeptide comprenant ou consistant en un ou plusieurs fragment(s) de flagelline, ledit polypeptide gardant sa capacité à lier et activer le récepteur 5 de type Toll (TLR5). Au sens de la présente demande, le terme « récepteur 5 de type Toll » ou « TLR5 » désigne un TLR5 de n'importe quelle espèce, de préférence un TLR5 humain. Selon un mode de réalisation, un polypeptide de flagelline tel que décrit ici comprend les domaines de la flagelline impliqués dans la signalisation TLR5. Le terme « domaine de la flagelline » inclut les domaines d'origine naturelle de la flagelline et les variants conservatifs fonctionnels de ceux-ci. Les domaines de flagelline impliqués dans la signalisation TLR5 sont bien connus de l'homme du métier, voir par exemple Smith et al. (2003) Nat. Immunol. 4: 1247-1253 (*e.g.,* acides aminés 78-129, 135-173 et 394-444 de la flagelline de *S. typhimurium* ou des homologues ou formes modifiés de celle-ci).
- "Réinfection" ou "infection secondaire" ou "seconde infection" : désigne une nouvelle infection d'un organisme par un agent infectieux ayant provoqué une primo-infection (la primo-infection étant la première atteinte d'un organisme par un agent infectieux ayant été traitée par la combinaison de la présente invention) ou par un agent infectieux de la même espèce que celui ayant provoqué la primo-infection. Les réinfections peuvent être répétées.
- "Souche probiotique" : désigne un microorganisme vivant qui, administré en quantité efficace par un hôte, lui confère un bénéfice en matière de santé. Parmi les probiotiques, on distingue en particulier les levures et les bactéries.
- "Sujet" : désigne un mammifère, de préférence un humain. Dans un mode de réalisation, le sujet peut être un « patient », *i.e.* un animal à sang chaud, de préférence un humain, en attente de recevoir ou recevant des soins médicaux, qui a fait l'objet d'une procédure médicale, ou qui est suivi pour le développement d'une infection respiratoire. Dans un autre mode de réalisation, le sujet est un animal de sang chaud, de préférence un humain, qui ne présente pas d'infection respiratoire.
- "Traitement" ou "traiter" : désigne à la fois le traitement thérapeutique et les mesures prophylactiques ou préventives, dont l'objet est d'inhiber ou détruire l'agent infectieux responsable d'une infection respiratoire et/ou de prévenir l'apparition d'une infection respiratoire ou sa réapparition. Les sujets ayant besoin d'un traitement incluent les sujets ayant déjà une infection respiratoire, les sujets à risque de développer une infection respiratoire et les sujets chez qui une infection respiratoire doit être prévenue. Selon un mode de réalisation, un sujet est considéré comme traité avec succès pour une infection respiratoire si, après avoir reçu une quantité thérapeutiquement efficace de la combinaison de la présente invention, le sujet montre, de façon observable ou mesurable, une réduction du nombre d'agents infectieux (responsable de l'infection respiratoire), une réduction des symptômes, une réduction de la morbidité et/ou une amélioration de la qualité de vie. Selon un mode de réalisation, le sujet est considéré comme traité si, après avoir reçu une quantité thérapeutiquement efficace de la combinaison de la présente invention, ledit sujet ne développe pas d'infection respiratoire suite à la mise en contact avec l'agent infectieux ciblé par ladite combinaison ou développe une infection respiratoire moins sévère qu'une infection généralement observée en l'absence de traitement. Les paramètres d'évaluation ci-dessus sont facilement mesurables par les procédures de routine familières à un médecin.
- "Unibody" : désigne un fragment d'anticorps issu des anticorps IgG4, dans lequel la région charnière a été enlevée. La suppression de la région charnière permet d'obtenir une molécule de la moitié de la taille d'un anticorps IgG4 classique, et qui présente un domaine de liaison monovalent contrairement au domaine de liaison bivalent des anticorps IgG4.
- « Variants de flagelline » : désigne des variants conservatifs fonctionnels, c'est-à-dire des variants dans lesquels un ou plusieurs résidu(s) d'acide aminé donné a/ont été modifié(s) sans altérer la conformation globale et la fonction de la flagelline, ce qui inclut, de façon non-limitative, le remplacement d'un acide aminé par un autre ayant des propriétés similaires (telles que, par exemple, la polarité, le potentiel de liaison hydrogène, l'acidité, la basicité, l'hydrophobie, l'aromaticité et autres).
- "Versabody" : désigne une protéine artificielle se liant à un antigène. C'est une petite protéine de 3 à 5 kDa avec plus de 15% de cystéines, qui forme un échafaudage disulfure de haute densité, remplaçant ainsi le cœur hydrophobique typique des protéines.
- "Virus" : désigne un agent infectieux qui ne peut se répliquer qu'en pénétrant dans une cellule vivante et en utilisant sa machinerie cellulaire. Un virus est composé d'un génome viral éventuellement enveloppé dans une coque protéique ou capside, voire d'une enveloppe. Le génome viral peut être un simple ou un double brin d'ADN ou d'ARN.

### DESCRIPTION DETAILLÉE

La présente invention concerne une combinaison d'au moins un agent capable de lier un agent infectieux respiratoire et d'au moins un agent immunomodulateur qui est un immunostimulant pour son utilisation dans le traitement ou la prévention à long terme d'une infection respiratoire et d'une réinfection respiratoire chez un sujet, l'au moins un agent capable de lier l'agent infectieux respiratoire étant à administrer (ou étant formulé pour administration) par inhalation chez le sujet, étant entendu par long-terme que le traitement ou la prévention perdure alors que l'au moins un agent capable de lier l'agent infectieux n'est plus détecté dans l'organisme et/ou lorsqu'une réponse immunitaire adaptative est induite dans l'organisme.

L'au moins un agent capable de lier l'agent infectieux respiratoire est un anticorps, un dérivé d'anticorps (par exemple un fragment d'anticorps) ou un mimétique d'anticorps. Il est en effet choisi parmi un anticorps, un anticorps bispécifique, un anticorps multispécifique, un fragment d'anticorps, un anticorps à domaine unique, un unibody, un nanobody, un affibody, une affiline, une affitine, une adnectine, une atrimère, une DARPin, une anticaline, une avimère, une fynomère ou un versabody.

Selon un mode de réalisation, la combinaison de la présente invention comprend un agent immunostimulant. Selon un mode de réalisation, la combinaison de la présente invention comprend plusieurs agents immunostimulants.

Selon un mode de réalisation, l'au moins un agent immunostimulant est une souche probiotique ou un mélange de souches probiotiques. Selon un mode de réalisation, l'agent immunostimulant est un mélange de 2, 3 ou 4 souches probiotiques, préférentiellement un mélange de 2 souches probiotiques.

Selon un mode de réalisation, la ou les souches probiotiques sont des bactéries.

Selon un mode de réalisation, la ou les souches probiotiques sont des bactéries vivantes ou actives, *i.e.* capables de se multiplier.

Selon un autre mode de réalisation, la ou les souches probiotiques sont des bactéries inactives, *i.e.* ne pouvant pas se multiplier.

Au sens de la présente invention, la ou les souches probiotiques sont dites vivantes ou actives si les bactéries sont capables de se multiplier dans des conditions de culture adaptées pour la croissance des dites bactéries.

Selon un mode de réalisation, la ou les souches probiotiques sont des bactéries sélectionnées parmi les bactéries Gram positives. Selon un mode de réalisation, la ou les souches probiotiques sont des bactéries sélectionnées parmi les bactéries Gram négatives.

Des exemples de bactéries Gram positives et Gram négatives pouvant être utilisées dans la présent invention incluent de façon non limitative, la famille des *Lactobacillaceae,* la famille des *Enterococcaceae,* la famille des *Bifidobacteriaceae* et la famille des *Enterobacteriaceae.*

Selon un mode de réalisation, l'au moins un agent immunostimulateur est ou comprend une souche probiotique ou un mélange de souches probiotiques sélectionnée(s) parmi la famille *Lactobacillaceae.*

Selon un mode de réalisation, l'au moins un agent immunostimulant est ou comprend une souche probiotique ou un mélange de souches probiotiques sélectionnée(s) parmi la famille *Bifidobacteriaceae.*

Selon un mode de réalisation, l'au moins un agent immunostimulant est ou comprend une souche probiotique ou un mélange de souches probiotiques sélectionnée(s) parmi la famille *Enterococcaceae,* préférentiellement parmi l'espèce *Enterococcus faecalis.*

Selon un mode de réalisation, l'au moins un agent immunostimulant est ou comprend une souche probiotique ou un mélange de souches probiotiques sélectionnée(s) parmi la famille *Enterobacteriaceae,* préférentiellement parmi l'espèce *Escherichia coli.*

Selon un mode de réalisation, l'au moins un agent immunostimulant est ou comprend une souche probiotique ou un mélange de souches probiotiques sélectionnée(s) parmi le genre *Lactobacillus.*

Selon un mode de réalisation, l'au moins un agent immunostimulant est ou comprend une souche probiotique sélectionnée parmi l'espèce *Lactobacillus rhamnosus* et/ou une espèce proche en terme génomique.

Selon un mode de réalisation, l'au moins un agent immunostimulant est ou comprend une souche probiotique sélectionnée parmi l'espèce *Lactobacillus salivarius* et/ou une espèce proche en terme génomique.

Selon un mode de réalisation, l'au moins un agent immunostimulant est ou comprend un mélange de souches probiotiques sélectionnées parmi les espèces *Lactobacillus rhamnosus* et *Lactobacillus salivarius.*

Selon un mode de réalisation, l'au moins un agent immunostimulant est un mélange d'une souche probiotique sélectionnée parmi l'espèce *Lactobacillus rhamnosus* (et/ou une espèce proche en terme génomique) et d'une souche probiotique sélectionnée parmi l'espèce *Lactobacillus salivarius* (et/ou une espèce proche en terme génomique).

Selon un mode de réalisation, une espèce proche de *Lactobacillus rhamnosus et Lactobacillus salivarius* en terme génomique est une espèce dont l'ARN ribosomique 16S présente au moins 97% (par exemple environ 98%, 99% ou plus de 99%) d'homologie de séquence avec l'ARN ribosomique 16S des espèces *Lactobacillus rhamnosus et Lactobacillus salivarius* respectivement.

Selon un mode de réalisation, une espèce proche de *Lactobacillus rhamnosus et Lactobacillus salivarius* en terme génomique est une espèce dont le génome total présente au moins 97% (par exemple environ 98%, 99% ou plus de 99%) d'homologie de séquence avec le génome complet des espèces *Lactobacillus rhamnosus* et *Lactobacillus salivarius* respectivement.

Selon un mode de réalisation, l'au moins un agent immunostimulant est ou comprend une souche probiotique ou un mélange de souches probiotiques sélectionnée(s) parmi l'espèce *Lactobacillus murinus* et/ou une espèce proche en terme génomique.

Des exemples de souches probiotiques de l'espèce *Lactobacillus murinus* incluent, de façon non limitative, la souche 313^{T} et la souche CNCM I-5314 déposée à la CNCM le 16 Avril 2018 au nom de Institut National de la Recherche Agronomique (devenu Institut National de Recherche pour l'Agriculture, l'Alimentation et l'Environnement en date du 1^{er} janvier 2020).

La souche 313^{T} est décrite dans Zheng et al. (« A taxonomic note on the genus Lactobacillus: Description of 23 novel genera, emended description of the genus Lactobacillus Beijerinck 1901 and union of Lactobacillaceae and Leuconostocaceae », Int J Syst Evol Microbiol, 2020, 70(4): 2782-2858). Elle est disponible à l'ATCC (American Type Culture Collection) sous la référence 35020^{T}, à la CCUG (Culture Collection University of Gothenburg) sous la référence 33904^{T}, à la CIP (Collection de l'institut Pasteur) sous la référence 104818^{T}, à la CNRZ (Centre national de recherches zootechniques) sous la référence 220^{T}, à la DSMZ (German Collection of Microorganisms and Cell Cultures) sous la référence 20452^{T}, à la NBRC (NITE Biological Resource Center) sous la référence 14221^{T}, à la JCM (Japan Collection of Microorganisms) sous la référence 1717^{T} et à la LMG (Belgian Coordinated Collections of Micro-organisms ) sous la référence 14189^{T}.

La souche CNCM I-5314 est décrite dans Bernard-Raichon L. et al. ("A Pulmonary Lactobacillus murinus Strain Induces Th17 and RORγt+ Regulatory T Cells and Reduces Lung Inflammation in Tuberculosis", 2021 Sep 3:ji2001044).

De plus, des analyses de séquences par séquençage Illumina NGS ont montré que les souches CNCM I-4967 et CNCM I-4968, déposées à la CNCM le 14 avril 2015 au nom de Institut National de la Recherche Agronomique (devenu Institut National de Recherche pour l'Agriculture, l'Alimentation et l'Environnement en date du 1er janvier 2020) appartiennent également à l'espèce *Lactobacillus murinus.*

Selon un mode de réalisation, une espèce proche de *Lactobacillus murinus* en terme génomique est une espèce dont l'ARN ribosomique 16S présente au moins 97% (par exemple environ 98%, 99% ou plus de 99%) d'homologie de séquence avec l'ARN ribosomique 16S de l'espèce *Lactobacillus murinus.*

Selon un mode de réalisation, une espèce proche de *Lactobacillus murinus* en terme génomique est une espèce dont le génome total présente au moins 97% (par exemple environ 98%, 99% ou plus de 99%) d'homologie de séquence avec le génome complet de l'espèce *Lactobacillus murinus.*

Les dénominations utilisées ci-dessus renvoient à la nomenclature antérieure à 2020 des *Lactobacillaceae,* une nouvelle classification ayant été proposée récemment (Zheng et al, « A taxonomic note on the genus Lactobacillus : Description of 23 novel genera, emended description of the genus Lactobacillus Beijerinck 1901 and union of Lactobacillaceae and Leuconostocaceae », Int J Syst Evol Microbiol, 2020, 70(4): 2782-2858). L'homme du métier est à même de faire la correspondance entre une ancienne et une nouvelle taxonomie. Si la taxonomie devait à nouveau être modifiée, l'homme du métier pourrait adapter les modifications de taxonomie pour en déduire les bactéries désignées dans la présente invention.

Ainsi, à titre d'exemples, les dénominations *Lactobacillus rhamnosus* et *Lactobacillus salivarius* utilisées dans l'ancienne nomenclature correspondent respectivement à *Lacticaseibacillus rhamnosus* et *Ligilactobacillus salivarius* de la nouvelle nomenclature. En outre, la dénomination *Lactobacillus murinus* utilisée dans l'ancienne nomenclature correspond à *Ligilactobacillus murinus* de la nouvelle nomenclature.

Selon un mode de réalisation, l'au moins un agent immunostimulant est ou comprend la souche probiotique déposée à la CNCM le 14 avril 2015 sous le numéro CNCM I-4967, au nom de Institut National de la Recherche Agronomique (devenu Institut National de Recherche pour l'Agriculture, l'Alimentation et l'Environnement en date du 1^{er} janvier 2020).

Selon un mode de réalisation, l'au moins un agent immunostimulant est ou comprend la souche probiotique déposée à la CNCM le 14 avril 2015 sous le numéro CNCM I-4968 au nom de Institut National de la Recherche Agronomique (devenu Institut National de Recherche pour l'Agriculture, l'Alimentation et l'Environnement en date du 1^{er} janvier 2020).

Selon un mode de réalisation, l'au moins un agent immunostimulant est ou comprend la souche probiotique déposée à la CNCM le 16 Avril 2018 sous le numéro CNCM I-5314 au nom de Institut National de la Recherche Agronomique (devenu Institut National de Recherche pour l'Agriculture, l'Alimentation et l'Environnement en date du 1^{er} janvier 2020).

Selon un mode de réalisation particulier, l'au moins un agent immunostimulant de la présente invention est ou comprend un mélange des 2 souches probiotiques déposées à la CNCM le 14 avril 2015 sous les numéros CNCM I-4967 et CNCM I-4968. Selon un mode de réalisation particulier, l'au moins un agent immunostimulant est ou comprend un mélange de la souche probiotique déposée à la CNCM le 14 Avril 2015 sous le numéro CNCM I-4967 et de la souche probiotique déposée à la CNCM le 16 Avril 2018 sous le numéro CNCM I-5314. Selon un mode de réalisation, l'au moins un agent immunostimulant est ou comprend un mélange de la souche probiotique déposée à la CNCM le 14 Avril 2015 sous le numéro CNCM I-4968 et de la souche probiotique déposée à la CNCM le 16 Avril 2018 sous le numéro CNCM I-5314.

Selon un mode de réalisation particulier, l'au moins un agent immunostimulant de la présente invention est ou comprend un mélange des 3 souches probiotiques déposées à la CNCM le 14 avril 2015 sous les numéros CNCM I-4967 et CNCM I-4968 et le 16 Avril 2018 sous le numéro CNCM I-5314.

Selon un mode de réalisation, l'au moins un agent immunostimulant est ou comprend une molécule chimique, une molécule peptidique, une molécule protéique, un sucre, un lipide ou un acide nucléique codant un agent immunostimulant.

Selon un mode de réalisation, l'au moins un agent immunostimulant est ou comprend une molécule se fixant sur les récepteurs de reconnaissance de motifs moléculaires (PRR, Pattern-Recognition Receptors).

Selon un mode de réalisation, la molécule se fixant sur les récepteurs de reconnaissance de motifs moléculaires est sélectionnée parmi les agonistes des récepteurs de type Toll (TLR, Toll-like receptors) ; les agonistes des récepteurs de type NOD (NOD-like receptors) et les agonistes des récepteurs de type RIG (RIG-like receptors).

Des exemples d'agonistes de ces récepteurs pouvant être utilisés dans le cadre de la présente invention incluent de façon non limitative, les diacyl et triacyl de lipopeptides, le peptidoglycane, les lipoprotéines, l'ARN simple et double brin, le lipopolysaccharide (LPS), la flagelline, l'ADN CpG non-méthylé, l'acide Polyinosinique-polycytidylique (poly I:C) et les acides lipotéichoïques. Un autre exemple d'agonistes de ces récepteurs est un polypeptide comprenant un ou plusieurs fragment(s) de flagelline (« polypeptide de flagelline »), ou un variant de flagelline.

Selon un mode de réalisation, l'au moins un agent capable de lier l'agent infectieux respiratoire est combiné avec une ou plusieurs molécules se fixant sur les récepteurs de reconnaissance de motifs moléculaires et une ou plusieurs souches probiotiques.

Selon un mode de réalisation, l'au moins un agent immunostimulant est ou comprend une molécule se fixant sur les récepteurs à la surface des cellules et modulant la réponse immunitaire, telle que les cytokines, chémokines et similaires.

Selon un mode de réalisation, l'au moins un agent immunostimulant est ou comprend une cytokine ou un mélange de cytokines. Des exemples de cytokines pouvant être utilisées dans le cadre de la présente invention incluent de façon non limitative, l'interleukine (IL)-1b, l'IL-6, l'IL-7, l'IL-12, l'IL-15, l'IL-17, l'IL-23, le Facteur de Nécrose Tumorale α (TNF α), l'interféron de type I (IFN type I).

Selon un mode de réalisation, l'au moins un agent capable de lier l'agent infectieux respiratoire est combiné avec une ou plusieurs cytokines et une ou plusieurs souches probiotiques.

Selon un mode de réalisation, l'au moins un agent immunostimulant est ou comprend une chémokine ou un mélange de chémokines. Des exemples de chémokines pouvant être utilisées dans le cadre de la présente invention incluent de façon non limitative, la CCL5 (Chemokine C-C motif ligand 5), la CCL27 (Chemokine C-C motif ligand 27), la CXCL9 (Chemokine C-X-C motif ligand 9), la CXCL10 (Chemokine C-X-C motif ligand 10) et la CXCL11 (Chemokine C-X-C motif ligand 11), CXCL16 (Chemokine C-X-C motif ligand 10).

Selon un mode de réalisation, l'au moins un agent capable de lier l'agent infectieux respiratoire est combiné avec une ou plusieurs chémokines et une ou plusieurs souches probiotiques.

Selon un mode de réalisation, l'au moins un agent immunostimulant est ou comprend un adjuvant, par exemple le chitosan.

Selon un mode de réalisation, l'au moins un agent capable de lier l'agent infectieux respiratoire est combiné avec un ou plusieurs adjuvants et une ou plusieurs souches probiotiques.

Selon un mode de réalisation, l'au moins un agent capable de lier l'agent infectieux respiratoire est combiné avec le chitosan et une ou plusieurs souches probiotiques.

Des exemples d'agents immunostimulants pouvant être utilisés dans la présente invention incluent de façon non limitative, la flagelline, un oligodésoxynucléotide CpG (CpG ODN), l'α-galactosylcéramide (α-Gal-Cer), les sels d'aluminium (hydroxyde d'aluminium, phosphate d'aluminium, et sulfate de potassium et d'aluminium), le MF59, le AS03, l'acide polyinosinique-polycytidylique et un polyphosphazène. Un autre exemple d'agent immunostimulant pouvant être utilisé dans la présente invention est un polypeptide comprenant ou consistant en un ou plusieurs fragment(s) de flagelline (qui peut être désigné « polypeptide de flagelline ») ou un variant de flagelline.

Selon un mode de réalisation, l'au moins un agent capable de lier l'agent infectieux respiratoire est combiné avec la flagelline et une ou plusieurs souches probiotiques. Selon un mode de réalisation, l'au moins un agent capable de lier l'agent infectieux respiratoire est combiné avec un polypeptide comprenant ou consistant en un ou plusieurs fragment(s) de flagelline (*i.e.* « polypeptide de flagelline ») et une ou plusieurs souches probiotiques. Selon un mode de réalisation, l'au moins un agent capable de lier l'agent infectieux respiratoire est combiné avec un variant de flagelline et une ou plusieurs souches probiotiques.

Des exemples de flagellines, variants de flagelline ou polypeptides de flagelline incluent, de façon non limitative, les peptides décrits dans les brevets US6,585,980; US6,130,082; US5,888,810; US5,618,533; et US4,886,748 et les demandes de brevet US2003/0044429, WO2008097016 et WO2009156405, qui sont incorporés par référence dans la présente demande.

Des exemples de flagellines incluent, de façon non limitative, des peptides comprenant ou consistant en la séquence SEQ ID NO : 10 (flagelline *E. coli* O157:H7), la séquence SEQ ID NO : 11 (flagelline de *S*. *typhimurium*) ou la séquence SEQ ID NO : 12 (flagelline de *S*. *typhimurium*).
SEQ ID NO : 10 
MAQVINTNSLSLITQNNINKNQSALSSSIERLSSGLRINSAKDDAAGQAIANRFTS
NIKGLTQAARNANDGISVAQTTEGALSEINNNLQRIRELTVQATTGTNSDSDLD
SIQDEIKSRLDEIDRVSGQTQFNGVNVLAKDGSMKIQVGANDGETITIDLKKIDS
DTLGLNGFNVNGKGTITNKAATVSDLTSAGAKLNTTTGLYDLKTENTLLTTDA
AFDKLGNGDKVTVGGVDYTYNAKSGDFTTTKSTAGTGVDAAAQAADSASKR
DALAATLHADVGKSVNGSYTTKDGTVSFETDSAGNITIGGSQAYVDDAGNLTT
NNAGSAAKADMKALLKAASEGSDGASLTFNGTEYTIAKATPATTTPVAPLIPG
GITYQATVSKDVVLSETKAAAATSSITFNSGVLSKTIGFTAGESSDAAKSYVDD
KGGITNVADYTVSYSVNKDNGSVTVAGYASATDTNKDYAPAIGTAVNVNSAG
KITTETTSAGSATTNPLAALDDAISSIDKFRSSLGAIQNRLDSAVTNLNNTTTNLS
EAQSRIQDADYATEVSNMSKAQIIQQAGNSVLAKANQVPQQVLSLLQG
SEQ ID NO : 11
SEQ ID NO: 12

AQVINTNSLSLLTQNNLNKSQSALGTAIERLSSGLRINSAKDDAAGQAIANRFTA
NIKGLTQASRNANDGISIAQTTEGALNEINNNLQRVRELAVQSANSTNSQSDLD

SIQAEITQRLNEIDRVSGQTQFNGVKVLAQDNTLTIQVGANDGETIDIDLKQINS
QTLGLDTLNVQQKYKVSDTAATVTGYADTTIALDNSTFKASATGLGGTDQKID
GDLKFDDTTGKYYAKVTVTGGTGKDGYYEVSVDKTNGEVTLAGGATSPLTGG
LPATATEDVKNVQVANADLTEAKAALTAAGVTGTASVVKMSYTDNNGKTIDG
GLAVKVGDDYYSATQNKDGSISINTTKYTADDGTSKTALNKLGGADGKTEVV
SIGGKTYAASKAEGHNFKAQPDLAEAAATTTENPLQKIDAALAQVDTLRSDLG
AVQNRFNSAITNLGNTVNNLTSARSRIEDSDYATEVSNMSRAQILQQAGTSVLA
QANQVPQNVLSLLR

Ainsi, selon un mode de réalisation, la flagelline est un peptide de séquence SEQ ID NO : 10. Selon un mode de réalisation, la flagelline est un peptide de séquence SEQ ID NO : 11. Selon un mode de réalisation, la flagelline est un peptide de séquence SEQ ID NO : 12. Ces flagellines sont décrites dans la demande internationale WO2016102536.

Selon un mode de réalisation, la flagelline ou un variant de flagelline présente au moins 70, 75, 80, 85, 90, 95, 97, 98 ou 99% d'identité avec l'une des séquences SEQ ID Nos : 10-12.

Selon un mode de réalisation, la flagelline ou un variant de flagelline comprend les résidus d'acides aminés 89-96 de SEQ ID NO : 12 (*i.e.* résidus impliqués dans la détection de TLR5). Ainsi, selon un mode de réalisation, la flagelline ou un variant de flagelline comprend la séquence SEQ ID NO : 15.
SEQ ID NO : 15 = QRVRELAV

Des exemples de polypeptides de flagelline incluent, de façon non limitative, les polypeptides décrits dans les demandes WO2009156405 et WO2016102536, qui sont incorporés par référence dans la demande.

Selon un mode de réalisation, le polypeptide de flagelline comprend :
a) un peptide N-terminale ayant au moins 90% d'identité avec la séquence d'acides aminés commençant par le résidu d'acides aminés localisé à la position de 1 de SEQ ID NO : 12 et terminant par un résidu d'acides aminés sélectionné parmi le groupe consistant en n'importe lequel des résidus d'acides aminés localisés aux positions 99 à 173 de SEQ ID NO : 12 et
b) un peptide C-terminale ayant au moins 90% d'identité avec la séquence d'acides aminés commençant par un résidu d'acides aminés sélectionné parmi le groupe consistant en n'importe lequel des résidus d'acides aminés localisés aux positions 401 à 406 de SEQ ID NO : 12 et terminant par le résidu d'acides aminés localisé à la position 494 de SEQ ID NO : 12, dans lequel : ledit peptide N-terminale est directement lié audit peptide C-terminale, ou ledit peptide N-terminale et ledit peptide C-terminale sont liés de façon indirecte l'un à l'autre, avec une chaine d'espacement.

Selon un mode de réalisation, ledit peptide N-terminale est sélectionné parmi le groupe comprenant ou consistant en les séquences d'acides aminés 1-99, 1-137, 1-160 et 1-173 de SEQ ID NO : 12.

Selon un mode de réalisation, ledit peptide C-terminale est sélectionné parmi le groupe comprenant ou consistant en les séquences d'acides aminés 401-494 et 406-494 de SEQ ID NO : 12.

Selon un mode de réalisation, lesdits peptides N-terminale et C-terminale comprennent ou consistent en les séquences d'acides aminés 1-173 et 401-494 de SEQ ID NO : 12, respectivement.

Selon un mode de réalisation, lesdits peptides N-terminale et C-terminale comprennent ou consistent en les séquences d'acides aminés 1-160 et 406-494 de SEQ ID NO : 12, respectivement.

Selon un mode de réalisation, lesdits peptides N-terminale et C-terminale comprennent ou consistent en les séquences d'acides aminés 1-137 et 406-494 de SEQ ID NO : 12, respectivement.

Selon un mode de réalisation, ledit peptide N-terminale et ledit peptide C-terminale sont liés indirectement l'un à l'autre avec une chaine d'espacement. Un exemple non limitatif de chaîne d'espacement est une séquence comprenant ou consistant en la séquence peptidique SEQ ID NO : 13.
SEQ ID NO : 13 = NH2-Gly-Ala-Ala-Gly-COOH

Selon un mode de réalisation, le résidu d'acide aminé asparagine localisé à la position 488 de SEQ ID NO : 12 est remplacé par une sérine.

Selon un mode de réalisation, le polypeptide de flagelline tel que décrit précédemment comprend un résidu additionnel de méthionine à la terminaison N-terminale.

Selon un mode de réalisation, le polypeptide de flagelline tel que décrit précédemment comprend un résidu additionnel de méthionine (M) et un résidu additionnel de lysine (L) à la terminaison N-terminale.

Selon un mode de réalisation, le polypeptide de flagelline comprend des peptides N-terminale et C-terminale consistant en les séquences 1-173 et 401-494 de SEQ ID NO : 12, lesdits peptides étant indirectement liés l'un à l'autre avec une chaine d'espacement consistant en la séquence peptidique SEQ ID NO : 13, ledit polypeptide comprenant un résidu additionnel de méthionine et un résidu additionnel de lysine à la terminaison N-terminale. Selon un mode de réalisation, le polypeptide de flagelline est de séquence SEQ ID NO : 14 (FLAMOD).

SEQ ID NO : 14

MKAQVINTNSLSLLTQNNLNKSQSALGTAIERLSSGLRINSAKDDAAGQAIANR
FTANIKGLTQASRNANDGISIAQTTEGALNEINNNLQRVRELAVQSANSTNSQS
DLDSIQAEITQRLNEIDRVSGQTQFNGVKVLAQDNTLTIQVGANDGETIDIDLKQ
INSQTLGLDTLNGAAGATTTENPLQKIDAALAQVDTLRSDLGAVQNRFNSAITN
LGNTVNNLTSARSRIEDSDYATEVSNMSRAQILQQAGTSVLAQANQVPQSVLSL
LR

Selon un mode de réalisation, l'au moins un agent capable de lier l'agent infectieux respiratoire est combiné avec un oligodésoxynucléotide CpG et une ou plusieurs souches probiotiques.

Selon un mode de réalisation, l'au moins un agent capable de lier l'agent infectieux respiratoire est combiné avec l'α-galactosylcéramide et une ou plusieurs souches probiotiques.

Selon un mode de réalisation, l'au moins un agent capable de lier l'agent infectieux respiratoire est combiné avec un ou des sels d'aluminium et une ou plusieurs souches probiotiques.

Selon un mode de réalisation, l'au moins un agent capable de lier l'agent infectieux respiratoire est combiné avec le MF59 et une ou plusieurs souches probiotiques.

Selon un mode de réalisation, l'au moins un agent capable de lier l'agent infectieux respiratoire est combiné avec le AS03 et une ou plusieurs souches probiotiques.

Selon un mode de réalisation, l'au moins un agent capable de lier l'agent infectieux respiratoire est combiné avec l'acide polyinosinique-polycytidylique et une ou plusieurs souches probiotiques.

Selon un mode de réalisation, l'au moins un agent capable de lier l'agent infectieux respiratoire est combiné avec un polyphosphazène et une ou plusieurs souches probiotiques.

Selon un mode de réalisation, l'au moins un agent immunostimulant est ou comprend un anticorps, préférentiellement un anticorps dirigé contre les points du contrôle du système immunitaire tels que CTLA-4, PD-1, PD-L1 ou CD137.

Selon un mode de réalisation, l'au moins un agent capable de lier l'agent infectieux respiratoire est combiné avec un anticorps dirigé contre les points du contrôle du système immunitaire tels que CTLA-4, PD-1, PD-L1 ou CD137 et une ou plusieurs souches probiotiques.

Selon un mode de réalisation, la combinaison de la présente invention comprend un agent capable de lier l'agent infectieux respiratoire.

Selon un mode de réalisation, la combinaison de la présente invention comprend plusieurs agents capables de lier un ou plusieurs agent(s) infectieux respiratoires, par exemple deux agents capables de lier un ou deux agent(s) infectieux respiratoires.

Selon un mode de réalisation, l'au moins un agent capable de lier l'agent infectieux respiratoire est dirigé contre l'agent infectieux responsable de l'infection respiratoire.

Selon un mode de réalisation, l'agent infectieux respiratoire est sélectionné parmi le groupe comprenant ou constitué des virus, bactéries, champignons et parasites.

Des exemples non limitatifs de virus responsables d'infections respiratoires incluent les virus influenza, rhinovirus, parainfluenza, virus respiratoire syncitial (VRS) et coronavirus. Des exemples de coronavirus inclut, de façon non limitative, le coronavirus du syndrome respiratoire aigu sévère (SARS-CoV) et le coronavirus 2 du syndrome respiratoire aigu sévère (SARS-CoV-2).

Selon un mode de réalisation, l'agent infectieux est un virus, préférentiellement un virus influenza, un VRS, SARS-CoV ou SARS-CoV-2. Selon un mode de réalisation, le virus est temporairement extracellulaire lors de l'infection.

Des exemples non limitatifs de bactéries responsables d'infections respiratoires incluent *Streptococcus pneumoniae, Haemophilus influenzae, Moraxella catarrhalis, Pseudomonas aeruginosa, Klebsiella pneumoniae, Staphylococcus aureus* et *Acinetobacter baumanii.*

Des exemples non limitatifs de champignons responsables d'infections respiratoires incluent *Pneumocystis jirovici, Cryptococcus neoformens, Aspergillus species* et *Histoplasma capsulatum capsulatum.*

Selon un mode de réalisation, l'agent infectieux respiratoire est au moins temporairement extracellulaire, *i.e.* présent ou détectable à l'extérieur de la cellule au moins temporairement lors de l'infection.

Selon un mode de réalisation, l'agent infectieux respiratoire est une bactérie. Selon un mode de réalisation, l'agent infectieux respiratoire est une bactérie extracellulaire, c'est-à-dire que lors de l'infection, la bactérie est présente ou détectée à l'extérieur des cellules.

Selon un mode de réalisation, l'agent infectieux respiratoire est une bactérie Gram positive. Selon un autre mode de réalisation, l'agent infectieux respiratoire de la présente invention est une bactérie Gram négative.

Selon un mode de réalisation, l'agent infectieux respiratoire est une bactérie résistante à un ou plusieurs antibiotiques.

Une liste non exhaustive d'antibiotiques inclut, les pénicillines (telles que l'amoxicilline, l'acide clavulanique, l'ampicilline ou la cloxacilline) ; les cyclines (telles que la doxycycline, la minocycline ou la tétracycline) ; les céphalosporines (telles que le céfadroxil, le céfixime, le cefpodoxime, le ceftriazone ou le céfuroxime) ; les carbapénèmes (tels que le doripénème, l'ertapénème, l'imipénème ou le méropénème) ; les aminosides (tels que la gentamicine, l'amikacine, la netilmicine, la tobramycine, l'isépamicine, la néomycine, la streptomycine ou la spectinomycine) ; les macrolides (tels que l'azithromycine, la clarithromycine, la roxithromycine ou la spiramycine) ; les fluoroquinolones (telles que la ciprofloxacine, la lévofloxacine, la moxifloxacine, la norfloxacine ou l'ofloxacine) ; ou la fosfomycine.

Selon un mode de réalisation, l'agent infectieux respiratoire est une bactérie sélectionnée parmi le groupe ESKAPE.

Au sens de la présente invention, le groupe ESKAPE regroupe 6 bactéries pathogènes multi-résistantes : *Enterococcus faecium, Staphylococcus aureus, Klebsiella pneumoniae, Acinetobacter baumannii, Pseudomonas aeruginosa* et *Enterobacter spp.*

Selon un mode de réalisation, l'agent infectieux respiratoire est une bactérie sélectionnée parmi le groupe comprenant ou consistant en *Pseudomonas aeruginosa, Klebsiella pneumoniae, Staphylococcus aureus et Acinetobacter baumanii.*

Selon un mode de réalisation, l'agent infectieux respiratoire est *Pseudomonas aeruginosa,*

Selon un mode de réalisation, l'agent infectieux respiratoire est *Pseudomonas aeruginosa* de sérotype O6, O11, O10, O2, ou O1.

Selon un mode de réalisation, l'agent infectieux respiratoire est la souche PA103.

Selon un mode de réalisation, l'au moins un agent capable de lier l'agent infectieux respiratoire utilisé dans la présente invention est dirigé contre une molécule présente à la surface de l'agent infectieux respiratoire, en particulier de la bactérie infectieuse.

Selon un mode de réalisation, l'au moins un agent capable de lier l'agent infectieux respiratoire est dirigé contre une molécule exprimée à la surface de *Pseudomonas aeruginosa.*

Selon un mode de réalisation, ladite molécule est une protéine d'un système de virulence.

Selon un mode de réalisation, ladite molécule est un récepteur membranaire.

Selon un mode de réalisation, ladite molécule est une protéine d'un système de sécrétion.

Selon un mode de réalisation, ladite molécule est une protéine du système de sécrétion de type III.

Selon un mode de réalisation, ladite molécule est pcrV.

Selon un mode de réalisation, ladite molécule est exprimée par une ou plusieurs souches de *Pseudomonas aeruginosa.* Selon un mode de réalisation, ladite molécule est exprimée par la souche PA103.

L'au moins un agent capable de lier l'agent infectieux respiratoire utilisé dans la présente invention est une molécule sélectionnée parmi le groupe comprenant ou consistant en un anticorps, un dérivé d'anticorps ou un mimétique d'anticorps.

Selon un mode de réalisation, l'au moins un agent capable de lier l'agent infectieux respiratoire est un anticorps.

Selon un mode de réalisation, l'au moins un agent capable de lier l'agent infectieux respiratoire est un dérivé d'anticorps.

Selon un mode de réalisation, un dérivé d'anticorps est un fragment d'anticorps capable de lier un antigène.

Des exemples de fragments d'anticorps incluent, sans y être limités, un fragment variable (Fv), un fragment variable à chaine unique (scFv), un fragment Fab, un fragment Fab', un fragment F(ab)'₂, un fragment Fd, un diabody, un triabody et un tétrabody.

Selon un mode de réalisation, le dérivé d'anticorps est un anticorps multispécifique, par exemple un anticorps bispécifique.

Selon un mode de réalisation, le dérivé d'anticorps est un anticorps à domaine unique, un unibody ou un nanobody.

Selon un mode de réalisation, l'au moins un agent capable de lier l'agent infectieux respiratoire est un mimétique d'anticorps.

Selon un mode de réalisation, le mimétique d'anticorps est couplé avec un fragment Fc d'anticorps.

Des exemples de mimétiques d'anticorps incluent les affibodies, les affilines, les affitines, les adnectines, les atrimères, les DARPins, les anticalines, les avimères, les fynomères et les versabodies.

Selon un mode de réalisation, l'au moins un agent capable de lier l'agent infectieux respiratoire est une affitine.

Selon un mode de réalisation, l'au moins un anticorps (ou dérivé d'anticorps) utilisé dans la présente invention est un anticorps chimérique, humanisé ou d'origine humaine (ou un dérivé de celui-ci).

Selon un mode de réalisation, l'au moins un anticorps (ou dérivé d'anticorps) utilisé dans la présente invention appartient à l'un des isotypes suivants : IgG, IgA, IgM, IgE ou IgD, préférentiellement IgG, IgA ou IgM.

Selon un mode de réalisation, l'au moins un anticorps (ou dérivé d'anticorps) utilisé dans la présente invention est un anticorps monoclonal (ou un dérivé de celui-ci). Selon un autre mode de réalisation, l'au moins un anticorps (ou dérivé d'anticorps) de la présente invention est un anticorps polyclonal (ou un dérivé de celui-ci).

Selon un mode de réalisation, l'au moins un anticorps (ou dérivé d'anticorps) utilisé dans la présente invention est dirigé contre pcrV (SEQ ID NO : 9), *i.e.* un anticorps (ou dérivé d'anticorps) anti-PcrV.

SEQ ID NO : 9
MEVRNLNAARELFLDELLAASAAPASAEQEELLALLRSERIVLAHAGQPLSEAQ
VLKALAWLLAANPSAPPGQGLEVLREVLQARRQPGAQWDLREFLVSAYFSLH
GRLDEDVIGVYKDVLQTQDGKRKALLDELKALTAELKVYSVIQSQINAALSAK
QGIRIDAGGIDLVDPTLYGYAVGDPRWKDSPEYALLSNLDTFSGKLSIKDFLSGS
PKQSGELKGLSDEYPFEKDNNPVGNFATTVSDRSRPLNDKVNEKTTLLNDTSSR
YNSAVEALNRFIQKYDSVLRDILSAI

Des exemples d'anticorps dirigés contre pcrV sont décrits dans les demandes WO2010091189, WO2013070615, WO2015196011, WO02064161, WO2020252029 (le contenu de chacune de ces demandes est incorporé par référence).

La demande WO2013070615 décrit un anticorps anti-PcrV comprenant un domaine variable de la chaine lourde (VH) comprenant les 3 régions déterminant la complémentarité (CDR) suivantes :
- VH-CDR1 : SYAMN (SEQ ID NO: 16),
- VH-CDR2 : AITISGITAYYTDSVKG (SEQ ID NO: 17),
- VH-CDR3 : EEFLPGTHYYYGMDV (SEQ ID NO : 18), et
un domaine variable de la chaine légère (VL) comprenant les 3 CDRs suivantes :
- VL-CDR1 : RASQGIRNDLG (SEQ ID NO: 19),
- VL-CDR2 : SASTLQS (SEQ ID NO: 20),
- VL-CDR3 : LQDYNYPWT (SEQ ID NO: 21).

La demande WO2020252029 décrit par exemple un anticorps anti-PcrV comprenant un domaine variable de la chaine lourde (VH) comprenant les 3 CDRs suivantes :
- VH-CDR1 : GFTFSSYA (SEQ ID NO : 22),
- VH-CDR2 : IRGSGYSS (SEQ ID NO: 23),
- VH-CDR3 : AKERSVTAYYYYGMDV (SEQ ID NO : 24), et
un domaine variable de la chaine légère (VL) comprenant les 3 CDRs suivantes :
- VL-CDR1 : QDISSF (SEQ ID NO: 25),
- VL-CDR2 : TAS, et
- VL-CDR3 : QQLKSYPLT (SEQ ID NO : 26).

La demande WO2020252029 décrit par exemple un anticorps anti-PcrV comprenant un domaine variable de la chaine lourde (VH) comprenant les 3 CDRs suivantes :
- VH-CDR1 : GFTFNTYA (SEQ ID NO : 27),
- VH-CDR2 : IGGSGYST (SEQ ID NO: 28),
- VH-CDR3 : AKEGNIVALYWYFDL (SEQ ID NO : 29), et
un domaine variable de la chaine légère (VL) comprenant les 3 CDRs suivantes :
- VL-CDR1 : QSISRY (SEQ ID NO : 30),
- VL-CDR2 : AAS, et
- VL-CDR3 : QQSSTTPLT (SEQ ID NO : 31).

La demande WO2020252029 décrit par exemple un anticorps anti-PcrV comprenant un domaine variable de la chaine lourde (VH) comprenant les 3 CDRs suivantes :
- VH-CDR1 : GFTFSDHE (SEQ ID NO : 32),
- VH-CDR2 : IGSGVVTM (SEQ ID NO : 33),
- VH-CDR3 : ARDRGYYFGSEAFHY (SEQ ID NO : 34), et
un domaine variable de la chaine légère (VL) comprenant les 3 CDRs suivantes :
- VL-CDR1 : QSISNW (SEQ ID NO : 35),
- VL-CDR2 : KSS, et
- VL-CDR3 : QQYKSYSLT (SEQ ID NO : 36).

La demande WO2020252029 décrit par exemple un anticorps anti-PcrV comprenant un domaine variable de la chaine lourde (VH) comprenant les 3 CDRs suivantes :
- VH-CDR1 : GFTFSTFA (SEQ ID NO : 37),
- VH-CDR2 : IGASGYST (SEQ ID NO : 38),
- VH-CDR3 : AKEYSVSSNYYYGMDV (SEQ ID NO : 39), et
un domaine variable de la chaine légère (VL) comprenant les 3 CDRs suivantes :
- VL-CDR1 : QTIRRY (SEQ ID NO : 40),
- VL-CDR2 : AAS, et
- VL-CDR3 : QQTYSIPIT (SEQ ID NO : 41).

Selon un mode de réalisation, l'au moins un anticorps (ou dérivé d'anticorps) utilisé dans la présente invention est un anticorps ayant des CDRs telles que décrites ci-dessus.

Selon un mode de réalisation, l'au moins un anticorps (ou dérivé d'anticorps) utilisé dans la présente invention est l'anticorps mAb166 (ou un dérivé de celui-ci). L'obtention et la caractérisation de l'anticorps mAb166 sont décrites dans l'art antérieur (Franck et al., Generation and characterization of a protective monoclonal antibody to Pseudomonas aeruginosa pcrV, The Journal of Infectious disease, July 2002; 186(1):64-73). L'anticorps mAb166 est disponible commercialement (CreativeBiolabs, Référence MRO-156MZ ; LGC Standards, Référence PTA-9180 hybridoma).

Selon un mode de réalisation, l'anticorps mAb166 comprend un domaine variable de la chaine lourde (VH) comprenant les 3 régions déterminant la complémentarité (CDR) suivantes :
- VH-CDR1 : SYGVH (SEQ ID NO : 1),
- VH-CDR2 : VIWSGGDTDYNAAFIS (SEQ ID NO : 2),
- VH-CDR3 : NRGDIYYDFTYAMDY (SEQ ID NO : 3), et
un domaine variable de la chaine légère (VL) comprenant les 3 CDRs suivantes :
- VL-CDR1 : RASGNIQNYLA (SEQ ID NO: 4),
- VL-CDR2 : SAKTLAD (SEQ ID NO: 5),
- VL-CDR3 : QHFWSTPYT (SEQ ID NO: 6).

Selon un mode de réalisation, les domaines variables des chaines lourdes et légères de l'anticorps mAb166 sont de séquences SEQ ID NO : 7 et SEQ ID NO : 8, respectivement.
SEQ ID NO : 7 
QVQLKQSGPGLVQPSQSLSITCTVSGFSLTSYGVHWVRQSPGKGLEWLGVIWS
GGDTDYNAAFISRLSISKDNSKSQLFFKMNSLRATDTAIYYCARNRGDIYYDFT
YAMDYWGQGTSVTVSS
SEQ ID NO : 8

DIQMTQSPASLSASVGETVTITCRASGNIQNYLAWYQQTQGKSPQLLVYSAKTL
ADGVPSRFSGSGSGTQYSLKINSLQPEDFGSYYCQHFWSTPYTFGGGTKLEIKR

Selon un mode de réalisation, l'au moins un anticorps (ou dérivé d'anticorps) utilisé dans la présente invention est un anticorps chimérique ou un anticorps humanisé issu de l'anticorps mAb166 (ou un dérivé de celui-ci).

Des méthodes d'humanisation d'anticorps sont bien connues de l'homme du métier.

Le choix des domaines variables humains des chaînes lourdes et légères à utiliser pour produire les anticorps humanisés est important pour réduire l'immunogénicité. Selon la méthode dite du « best-fit », la séquence du domaine variable de l'anticorps d'intérêt est comparée avec une librairie de séquences de domaines variables humains connus. La séquence humaine la plus proche de la séquence murine est ainsi sélectionnée comme la séquence humaine framework (FR) pour l'anticorps humanisé (Sims et al., J. Immunol. 151, pp. 2296 (1993); Chothia and Lesk, J. Mol. Biol. 196, pp. 901). Une autre méthode utilise une séquence FR particulière issue d'une séquence consensus de tous les anticorps humains d'un sous-groupe particulier des chaînes lourdes ou légères. La même séquence FR peut être utilisée pour plusieurs anticorps humanisés différents (Carter et al., PNAS 89, pp. 4285 (1992); Presta et al. J. Immunol., 151 (1993)).

Selon un mode de réalisation, l'au moins un anticorps (ou dérivé d'anticorps) utilisé dans la présente invention est un anticorps (ou un dérivé d'anticorps) se liant au même épitope que mAb166. Selon un mode de réalisation, l'au moins un anticorps (ou dérivé d'anticorps) utilisé dans la présente invention se lie à un fragment de pcrV comprenant les acides aminés 144 à 257 de pcrV (SEQ ID NO : 9).

Au sens de la présente invention, l'infection est dite respiratoire si elle atteint au moins l'une des structures composant le système respiratoire, *i.e.* le nez, la gorge, le larynx, la trachée, les bronches ou les poumons.

Selon un mode de réalisation, l'infection respiratoire est caractérisée par au moins l'un des symptômes suivants : toux sèche, toux grasse, fièvre modérée à forte, frissons, troubles de la respiration (par exemple essoufflements), présence d'un sifflement lors de la respiration, douleurs thoraciques, fatigue, écoulement nasal, perte temporaire du goût et de l'odorat.

Selon un mode de réalisation, l'infection respiratoire est causée par un agent infectieux respiratoire tel que décrit précédemment. Ainsi, selon un mode de réalisation, l'infection respiratoire est une infection à *Pseudomonas aeruginosa.*

Selon un mode de réalisation, l'infection respiratoire est une infection respiratoire des voies inférieures, *i.e.,* une infection respiratoire affectant les voies aériennes inférieures ou les poumons.

Selon un mode de réalisation, l'infection respiratoire est une infection respiratoire aigüe.

Des exemples non limitatifs d'infections respiratoires des voies inférieures incluent la bronchite, la bronchiolite, la pneumonie (par exemple une pneumopathie nosocomiale, une pneumopathie communautaire ou une pneumopathie acquise sous ventilation mécanique), la grippe et la coqueluche.

Ainsi, selon un mode de réalisation, l'infection respiratoire est une pneumonie (ou pneumopathie) nosocomiale.

Selon un mode de réalisation, l'infection respiratoire est une primo-infection (ou infection primaire), c'est-à-dire que le sujet n'a pas été préalablement traité par une combinaison de la présente invention pour une infection avec le même agent infectieux respiratoire ou avec un agent infectieux de la même espèce.

Selon un mode de réalisation, l'infection respiratoire est une réinfection (ou infection secondaire), par exemple la seconde réinfection, ou une réinfection répétée, *i.e.,* une nouvelle infection causée par le même agent infectieux respiratoire ou un agent infectieux respiratoire de la même espèce tel que défini précédemment.

Selon un mode de réalisation, l'infection respiratoire n'est pas une primo-infection au sens de la présente invention, c'est-à-dire que le sujet a préalablement été traité par une combinaison de la présente invention pour une infection avec le même agent infectieux respiratoire ou avec un agent infectieux respiratoire de la même espèce.

Selon un mode de réalisation, la combinaison de la présente invention traite une infection respiratoire chez un sujet.

Selon un mode de réalisation, la combinaison de la présente invention prévient l'apparition ou la réapparition d'une infection respiratoire chez un sujet.

La combinaison de la présente invention prévient à court terme l'apparition d'une infection respiratoire chez un sujet. Au sens de la présente invention, la prévention est dite à court-terme lorsque l'au moins un agent capable de lier l'agent infectieux respiratoire, préférentiellement un anticorps ou un dérivé d'anticorps, est encore détecté dans l'organisme après administration de la combinaison.

La combinaison de la présente invention induit un effet vaccinal (*i.e.* une protection ou prévention à long terme) prévenant l'apparition ou la réapparition d'une infection respiratoire chez un sujet. Au sens de la présente invention, la protection ou prévention est dite à long-terme lorsque la protection perdure alors que l'au moins un agent capable de lier l'agent infectieux respiratoire, préférentiellement un anticorps ou un dérivé d'anticorps, présent dans la combinaison de l'invention n'est plus détecté dans l'organisme et/ou lorsqu'une réponse immunitaire adaptative est induite dans l'organisme.

Selon un mode de réalisation, la protection ou prévention à long-terme dure au moins 60, au moins 70, au moins 80, au moins 90, ou au moins 100 jours après administration de la combinaison, préférentiellement au moins 100 jours.

Au sens de la présente invention, l'effet vaccinal est caractérisé par la capacité d'une combinaison telle que décrite ci-dessus à induire une réponse immunitaire adaptative permettant de prolonger son action au-delà de la disparition de l'au moins un agent capable de lier l'agent infectieux respiratoire, préférentiellement un anticorps ou un dérivé d'anticorps, présent dans la combinaison de l'organisme en stimulant des réponses immunitaires spécifiques.

Ainsi, selon un mode de réalisation, la combinaison de la présente invention induit une réponse immunitaire adaptative permettant de prévenir l'apparition ou la réapparition d'une infection respiratoire chez un sujet.

Ainsi, une des particularités de la présente invention est que l'administration de la combinaison de la présente invention prévient et/ou traite les primo-infections (effet à court-terme) mais aussi les réinfections (ou infections secondaires), éventuellement répétées (effet à long-terme) lorsque l'au moins un agent capable de lier l'agent infectieux, préférentiellement un anticorps ou un dérivé d'anticorps, présent dans la combinaison de l'invention n'est plus détecté dans l'organisme.

Selon un mode de réalisation, le sujet est considéré comme traité si le nombre d'agent infectieux chez ledit sujet est diminué après administration de la combinaison de la présente invention. Selon un mode de réalisation, le sujet est considéré comme traité si au moins un des symptômes de l'infection respiratoire diminue ou disparaît complètement. Selon un mode de réalisation, le sujet est considéré comme traité si la morbidité associée à l'infection respiratoire est réduite ou nulle.

Selon un mode de réalisation, le sujet est considéré comme traité si, après avoir reçu une quantité thérapeutiquement efficace de la combinaison de la présente invention, ledit sujet développe une infection respiratoire moins sévère qu'en l'absence de traitement au contact de l'agent infectieux ciblé par ladite combinaison.

Selon un mode de réalisation, le sujet est considéré comme traité si, après avoir reçu une quantité thérapeutiquement efficace de la combinaison de la présente invention, ledit sujet ne développe pas d'infection respiratoire au contact de l'agent infectieux ciblé par ladite combinaison.

Selon un mode de réalisation, le sujet est un mammifère, préférentiellement un humain.

Selon un mode de réalisation, le sujet est un homme. Selon un autre mode de réalisation, le sujet est une femme.

Selon un mode de réalisation, le sujet est un adulte (> 18 ans). Selon un autre mode de réalisation, le sujet est un enfant (< 18 ans).

Selon un mode de réalisation, le sujet souffre d'une infection respiratoire.

Selon un mode de réalisation, le sujet est à risque de développer une infection respiratoire.

Les facteurs de risque de développer une infection respiratoire incluent, sans y être limités, l'âge (enfants et personnes de plus de 65 ans), le tabagisme, l'abus d'alcool, l'usage de drogues, l'habitation d'un logement insalubre, la pollution, l'affection par une pathologie respiratoire chronique, une contamination aéroportée ou un contact avec un sujet malade et une infection nosocomiale (incluant par exemple la pneumopathie acquise sous ventilation mécanique et la pneumopathie nosocomiale).

Selon un mode de réalisation, le sujet souffre (de préférence est diagnostiqué comme souffrant) d'une pathologie respiratoire chronique.

Selon un mode de réalisation, ladite pathologie respiratoire chronique est sélectionnée parmi le groupe comprenant ou consistant en la bronchopneumopathie chronique obstructive (BPCO), les maladies interstitielles pulmonaires, le cancer du poumon, l'asthme (de l'adulte et de l'enfant), la bronchiectasie, les maladies rares et orphelines du poumon comme la mucoviscidose et les maladies vasculaires pulmonaires.

Selon un mode de réalisation, ladite pathologie respiratoire chronique est sélectionnée parmi le groupe comprenant ou consistant en la bronchopneumopathie chronique obstructive (BPCO), la bronchiectasie, les maladies rares et orphelines du poumon comme la mucoviscidose.

Des exemples non limitatifs de maladies interstitielle pulmonaires (aussi connues sous le nom de maladies pulmonaires parenchymateuses diffuses) incluent la sarcoïdose, la fibrose pulmonaire idiopathique (FPI), l'alvéolite allergique extrinsèque (pneumopathie d'hypersensibilité), la pneumopathie interstitielle associée à une maladie du tissu conjonctif, la pneumoconiose et la maladie interstitielle pulmonaire due à certains médicaments.

Des exemples non limitatifs de maladies rares et orphelines du poumon incluent la mucoviscidose, la lymphangioléiomyomatose (LAM), la sclérodermie, la protéinose alvéolaire pulmonaire (PAP), les maladies pulmonaires hyperéosinophiliques, le syndrome d'emphysème et fibrose pulmonaires combinés, les maladies kystiques multiples pulmonaires (MKMP), la dyskinésie ciliaire primitive pulmonaire, la pneumopathie organisée, les vascularites pulmonaires, les bronchiolites et BPCO non tabagiques, les syndromes hémorragiques alvéolaires, les syndromes lymphoprolifératifs pulmonaires primitifs, les trachéopathies, la télangiectasie hémorragique héréditaire avec malformation artérioveineuse pulmonaire (Maladie de Rendu-Osler) et l'amylose bronchopulmonaire.

Des exemples non limitatifs de maladies vasculaires pulmonaires incluent l'hypertension pulmonaire.

Selon un mode de réalisation, le sujet souffre d'une mucoviscidose.

Selon un mode de réalisation, le sujet souffre d'une bronchopneumopathie chronique obstructive (BPCO).

La présente invention concerne également une composition, une composition pharmaceutique ou un médicament pour son utilisation dans le traitement ou la prévention d'une infection respiratoire chez un sujet, ladite composition, ladite composition pharmaceutique ou ledit médicament comprenant ou consistant essentiellement en une combinaison telle que décrite précédemment.

Au sens de la présente invention, le terme « consiste essentiellement en », en référence à une composition, une composition pharmaceutique ou un médicament, signifie que l'objet de l'invention est le seul agent thérapeutique ou agent ayant une activité biologique dans ladite composition, composition pharmaceutique ou médicament.

Selon un mode de réalisation, la composition pharmaceutique pour son utilisation dans la présente invention comprend en outre au moins un excipient pharmaceutiquement acceptable.

Des exemples non limitatifs d'excipients liquides pharmaceutiquement acceptables incluent l'eau distillée, une solution saline, une solution aqueuse de glucose, de l'alcool par exemple de l'éthanol, du propylène glycol, et du polyéthylène glycol ; et des véhicules huileux tels que des huiles végétales et animales, de la paraffine, ou de la cire.

Des exemples non limitatifs d'excipients solides pharmaceutiquement acceptables incluent le glucose, le fructose, le sucrose, le maltose, la dextrine jaune, la dextrine blanche, la maltodextrine, la cellulose microcristalline, le stéarate de calcium, le stéarate de magnésium, le sorbitol, le sirop de glucose, le lactose, l'acide citrique, l'acide tartrique, l'acide malique, l'acide succinique, l'acide lactique, l'acide L-ascorbique, l'alpha-tocophérol, le glycérol, le propylène glycol, le sucroester, les esters poly glycériques d'acides gras, les sucroglycérides, les mono, di et triglycérides béhénate, les Carraghénane, la gomme arabique, la caséine, la gélatine, la pectine, l'agar, la nicotinamide, les acides aminés, les sels de calciums et les pigments.

La présente invention concerne également l'utilisation d'une combinaison telle que décrite précédemment pour la fabrication d'un médicament pour le traitement ou la prévention d'une infection respiratoire chez un sujet.

Selon un mode de réalisation, une dose d'environ 0.5 à 50 mg de l'au moins un agent capable de lier l'agent infectieux respiratoire de la présente invention, préférentiellement un anticorps ou un dérivé d'anticorps, par kilo de poids est à administrer (ou est pour administration) chez le sujet, préférentiellement une dose d'environ 1 à 20 mg par kilo, plus préférentiellement une dose d'environ 2.5 à 5 mg par kilo.

Selon un mode de réalisation, une souche probiotique telle que décrite précédemment est à administrer (ou est pour administration) chez le sujet à une quantité comprise entre 10³ et 10⁹ ufc (unité formant colonie).

Selon un mode de réalisation, chaque souche probiotique d'un mélange de 2 souches probiotiques tel que décrit précédemment est à administrer (ou est pour administration) chez le sujet à une quantité comprise entre 10³ et 10⁹ ufc. Selon un mode de réalisation, chaque souche probiotique d'un mélange de 3 souches probiotiques tel que décrit précédemment est à administrer (ou est pour administration) chez le sujet à une quantité comprise entre 10³ et 10⁹ ufc

Selon un mode de réalisation, un mélange de 2 souches probiotiques tel que décrit précédemment est à administrer (ou est pour administration) avec un ratio de 1:1, 1:2, 1:3, 1:4, 1:5, 1:6 ; 1:7, 1:8, 1:9 ou 1:10.

Selon un mode de réalisation, le chitosan tel que décrit précédemment est à administrer (ou est pour administration) à une dose d'environ 0.1 à environ 50 mg/kg, de préférence d'environ 0.5 à environ 20 mg par jour par kilo.

Selon un mode de réalisation, la flagelline, un variant de flagelline ou un polypeptide comprenant ou consistant en un ou plusieurs fragment(s) de flagelline (*i.e.* « polypeptide de flagelline ») tels que décrits précédemment sont à administrer (ou sont pour administration) à une dose d'environ 1 µg à 100 mg, préférentiellement à une dose d'environ 1 µg à 10 mg, préférentiellement à une dose d'environ 1 µg à 1 mg. Selon un mode de réalisation, la dose de flagelline, d'un variant de flagelline ou d'un polypeptide de flagelline est adaptée de façon à avoir localement une dose de 1 à 1000 µg d'une flagelline, d'un variant de flagelline ou d'un polypeptide de flagelline au niveau du tractus respiratoire.

Selon un mode de réalisation, l'au moins un agent capable de lier l'agent infectieux respiratoire, préférentiellement un anticorps ou un dérivé d'anticorps, et l'au moins un agent immunostimulant sont à administrer (ou sont pour administration) de manière simultanée.

Selon un autre mode de réalisation, l'au moins un agent capable de lier l'agent infectieux respiratoire, préférentiellement un anticorps ou un dérivé d'anticorps, et l'au moins un agent immunostimulant sont à administrer (ou sont pour administration) de manière séparée dans le temps.

Selon un mode de réalisation, l'au moins un agent immunostimulant est à administrer (ou est pour administration) avant l'au moins un agent capable de lier l'agent infectieux respiratoire, préférentiellement un anticorps ou un dérivé d'anticorps.

Selon un mode de réalisation, une souche probiotique ou un mélange de souches probiotiques telle(s) que décrite(s) précédemment est à administrer (ou est pour administration) avant l'au moins un agent capable de lier l'agent infectieux respiratoire, préférentiellement un anticorps ou un dérivé d'anticorps.

Selon un autre mode de réalisation, l'au moins un agent immunostimulant est à administrer (ou est pour administration) après l'au moins un agent capable de lier l'agent infectieux respiratoire, préférentiellement un anticorps ou un dérivé d'anticorps.

Selon un mode de réalisation, l'au moins un agent capable de lier l'agent infectieux respiratoire, préférentiellement un anticorps ou un dérivé d'anticorps, est à administrer (ou est pour administration) en une seule dose.

Selon un mode de réalisation, l'au moins un agent capable de lier l'agent infectieux respiratoire, préférentiellement un anticorps ou un dérivé d'anticorps, est à administrer (ou est pour administration) en doses répétées, par exemple une fois, deux fois ou trois fois.

Selon un mode de réalisation, l'au moins un agent immunostimulant est à administrer (ou est pour administration) en une seule dose.

Selon un mode de réalisation, l'au moins un agent immunostimulant, préférentiellement une souche probiotique ou un mélange de souches probiotiques, est à administrer (ou est pour administration) en doses répétées, par exemple une fois, deux fois ou trois fois.

Selon un mode de réalisation, l'au moins un agent immunostimulant, préférentiellement une souche probiotique ou un mélange de souches probiotiques, est à administrer (ou est pour administration) pendant au moins 1, 2, 3, 4, 5, 6, 7, 8, 9, 10, 11, 12, 13, 14 jours (préférentiellement des jours consécutifs) ou jusqu'à guérison complète du sujet.

Selon un mode de réalisation, l'au moins un agent capable de lier l'agent infectieux respiratoire, préférentiellement un anticorps ou un dérivé d'anticorps, et l'au moins un agent immunostimulant utilisé dans la présente invention (préférentiellement une souche probiotique ou un mélange de souches probiotiques), sont à administrer (ou sont pour administration) par des voies d'administration identiques, *i.e.* par inhalation.

Selon la présente invention, l'au moins un agent capable de lier l'agent infectieux respiratoire, préférentiellement un anticorps ou un dérivé d'anticorps, est sous une forme adaptée à une administration par inhalation. Selon un mode de réalisation, à la fois l'au moins un agent capable de lier l'agent infectieux respiratoire, préférentiellement un anticorps ou un dérivé d'anticorps, et l'au moins un agent immunostimulant sont sous une forme adaptée à une administration par inhalation.

Des exemples non limitatifs de formulations adaptées pour l'inhalation incluent les aérosols (liquides ou solides mis en suspension dans un gaz vecteur) et les sprays (liquides ou solides) à visée nasale.

Selon un autre mode de réalisation, l'au moins un agent capable de lier l'agent infectieux respiratoire, préférentiellement un anticorps ou un dérivé d'anticorps, et l'au moins un agent immunostimulant sont à administrer (ou sont pour administration) par des voies d'administration différentes.

Selon un mode de réalisation, l'au moins un agent immunostimulant est à administrer (ou est pour administration) par voie entérale, par voie topique ou par voie parentérale (voie injectable).

Au sens de la présente invention la voie entérale inclut la voie orale (voie entérale *per os*), la voie buccale, la voie sublinguale, la voie rectale, la voie pulmonaire, la voie percutanée, la voie nasale et les voies locales.

Au sens de la présente invention, une « administration orale » est une administration dans la cavité buccale, suivie par l'ingestion d'un composé, qui rejoint la circulation systémique à la suite de son absorption intestinale. Des exemples de formulations adaptées à une administration orale incluent, mais ne sont pas limités aux comprimés (dont les comprimés à libération prolongée), gélules, poudres, granulés, pilules (dont les pilules enrobées de sucre), capsules (dont les capsules de gélatine souple), suspensions orales, solutions buvables, et autres formes similaires.

Au sens de la présente invention, une « administration buccale » est une administration dans la cavité buccale d'un composé, qui n'est pas suivie de l'ingestion dudit composé, dont l'absorption se fait à travers les tissus buccaux, tels que, par exemple, le palais, le tissu sublingual, ou les gencives. Des exemples non limitatifs de formulations adaptées à une administration buccale incluent une gomme à mâcher, un patch et un spray buccal.

Au sens de la présente invention, une « administration rectale » est une administration d'un composé par l'anus, qui rejoint la circulation systémique à travers la muqueuse rectale. Des exemples non limitatifs de formulations adaptées à une administration rectale incluent les suppositoires, capsules rectales, lavements ou pommades.

Au sens de la présente invention, une « administration percutanée », ou « administration transdermique » est l'administration d'un composé sur la peau, suivie de son absorption dans la circulation sanguine systémique à travers les tissus cutanés adjacents. Des exemples non limitatifs de formulations adaptées à une administration transcutanée incluent la pommade, la pâte, l'onguent, le gel, la crème ou le patch transdermique.

Au sens de la présente invention, une « administration par voie nasale » est l'administration d'un composé directement sur la muqueuse nasale. Des exemples non limitatifs de formulations adaptées pour l'administration par voie nasale incluent les sprays, gouttes nasales, pommade nasale et solutions pour pulvérisation nasale.

Au sens de la présente invention, une « administration topique » est l'administration d'un composé sur une surface du corps, ledit composé n'ayant pas vocation à passer dans le système circulatoire. Des exemples de formulations adaptées à une administration topique incluent, mais ne sont pas limités aux compositions sous forme liquide, pâteuse, ou solide et, plus particulièrement, sous forme de solutions aqueuses, de collyres, de gouttes, de dispersions, de sprays, ou de microcapsules, micro- ou nanoparticules ou de patchs polymériques ou gélifiés permettant une libération contrôlée.

Au sens de la présente invention, le terme « voie injectable » inclut notamment les injections sous-cutanée, intraveineuse (IV), intramusculaire, intra-articulaire, intra-synovial, intracisternale, intrathécale, intrahépatique, intralésionnelle ou intra-crâniale. Des exemples de formulations adaptées à une administration par injection incluent, mais ne sont pas limités aux solutions aqueuses stériles, dispersions, émulsions, suspensions, formes solides appropriées pour la préparation de solutions ou suspensions par addition d'un liquide avant utilisation telles que, par exemple, des poudres.

Selon un mode de réalisation, l'au moins un agent immunostimulant comprend une ou plusieurs souches probiotiques et est à administrer (ou est pour administration) par inhalation, par voie orale (entérale *per os)* ou par voie nasale. Ainsi, selon ce mode de réalisation, l'au moins un agent immunostimulant est sous une forme adaptée à une administration par inhalation, par voie orale (entérale *per os*) ou par voie nasale.

La présente invention concerne également un nécessaire de pièces (pouvant également être dénommé kit) comprenant au moins deux parties, la première partie comprenant au moins un agent capable de lier l'agent infectieux respiratoire tel que décrit précédemment, ledit agent étant sélectionné parmi un anticorps, un anticorps bispécifique, un anticorps multispécifique, un fragment d'anticorps, un anticorps à domaine unique, un unibody, un nanobody, un affibody, une affiline, une affitine, une adnectine, une atrimère, une DARPin, une anticaline, une avimère, une fynomère et un versabody, et la seconde partie comprenant au moins un agent immunostimulant tel que décrit précédemment, pour son utilisation dans le traitement ou la prévention à long-terme d'une infection respiratoire et d'une réinfection respiratoire chez un sujet, dans lequel l'au moins un agent capable de lier l'agent infectieux respiratoire est à administrer par inhalation chez le sujet, étant entendu par long-terme que le traitement ou la prévention perdure alors que l'au moins un agent capable de lier l'agent infectieux n'est plus détecté dans l'organisme et/ou lorsqu'une réponse immunitaire adaptative est induite dans l'organisme..

Selon un mode de réalisation, ledit nécessaire de pièces comprend au moins un anticorps, au moins un dérivé d'anticorps ou au moins un mimétique d'anticorps tel que décrit précédemment et une souche probiotique telle que décrite précédemment. Selon un mode de réalisation, ledit nécessaire de pièces comprend au moins un anticorps ou un dérivé d'anticorps tel que décrit précédemment et un mélange de souches probiotiques tel que décrit précédemment. Selon un mode de réalisation, ledit nécessaire de pièces comprend au moins un anticorps dirigé contre *Pseudomonas aeruginosa* et un mélange des 2 souches probiotiques déposées le 14 avril 2015 à la CNCM sous les numéros CNCM I-4967 et CNCM I-4968.

Selon un mode de réalisation, ledit nécessaire de pièces comprend au moins un anticorps dirigé contre *Pseudomonas aeruginosa* et une souche probiotique sélectionnée parmi les souches déposées le 14 avril 2015 à la Collection Nationale de Cultures de Microorganismes (CNCM) sous les numéros CNCM I-4967 et CNCM I-4968 ou le 16 Avril 2018 sous le numéro CNCM 1-5314 ou un mélange de celles-ci.

Selon un mode de réalisation, la première partie du nécessaire de pièces comprend une composition pharmaceutique comprenant ou consistant essentiellement en au moins un agent capable de lier l'agent infectieux respiratoire tel que décrit précédemment, préférentiellement un anticorps ou un dérivé d'anticorps, et au moins un excipient pharmaceutiquement acceptable.

Selon un mode de réalisation, la seconde partie du nécessaire de pièces comprend une composition pharmaceutique comprenant ou consistant essentiellement en une ou plusieurs souches probiotiques telles que décrites précédemment, un ou plusieurs agonistes des récepteurs de type Toll, un ou plusieurs agonistes des récepteurs de type NOD, un ou plusieurs agonistes des récepteurs de type RIG, une cytokine ou un mélange de cytokines, une chémokine ou un mélange de chémokines, un ou plusieurs adjuvants tels que le chitosan, la flagelline, un oligodésoxynucléotide CpG (CpG ODN), l'α-galactosylcéramide (α-Gal-Cer), les sels d'aluminium (hydroxyde d'aluminium, phosphate d'aluminium, et sulfate de potassium et d'aluminium), le MF59, le AS03, l'acide polyinosinique-polycytidylique, un polyphosphazène ou un anticorps dirigé contre les points du contrôle du système immunitaire tels que CTLA-4, PD-1, PD-L1 ou CD137 et au moins un excipient pharmaceutiquement acceptable.

Selon un mode de réalisation, la seconde partie du nécessaire de pièces comprend une composition pharmaceutique comprenant ou consistant essentiellement en une flagelline, un variant de flagelline ou un polypeptide comprenant ou consistant en un ou plusieurs fragment(s) de flagelline (« polypeptide de flagelline ») tels que décrits précédemment et au moins un excipient pharmaceutiquement acceptable.

Des exemples d'excipients pharmaceutiquement acceptables sont donnés ci-dessus.

Selon un mode de réalisation, la première et la seconde partie dudit nécessaire de pièces sont à administrer (ou sont pour administration) simultanément ou de manière séparée dans le temps.

Selon un mode de réalisation, la seconde partie (comprenant au moins un agent immunostimulant tel que décrit ci-dessus) dudit nécessaire de pièces est à administrer (ou est pour administration) avant la première partie (comprenant au moins un agent capable de lier l'agent infectieux respiratoire tel que décrit ci-dessus).

Selon un mode de réalisation, la seconde partie (comprenant au moins un agent immunostimulant tel que décrit ci-dessus) dudit nécessaire de pièces est à administrer (ou est pour administration) après la première partie (comprenant au moins un agent capable de lier l'agent infectieux respiratoire tel que décrit ci-dessus).

Selon un mode de réalisation, la première et la seconde partie dudit nécessaire de pièces sont à administrer (ou sont pour administration) par des voies d'administration identiques. Selon ce mode de réalisation, la première et la seconde partie dudit nécessaire de pièces sont à administrer (ou sont pour administration) par inhalation.

Selon un mode de réalisation, la première et la seconde partie dudit nécessaire de pièces sont à administrer (ou sont pour administration) par des voies d'administration différentes. Selon ce mode de réalisation, la première partie dudit nécessaire de pièces (comprenant au moins un agent capable de lier l'agent infectieux respiratoire tel que décrit ci-dessus) est à administrer (ou est pour administration) par inhalation, et la seconde partie (comprenant au moins un agent immunostimulant tel que décrit ci-dessus) est à administrer (ou est pour administration) par une autre voie.

Selon la présente invention, la première partie dudit nécessaire de pièces est adaptée à une administration par inhalation. Selon un mode de réalisation, la première et la seconde partie dudit nécessaire de pièces sont adaptées à une administration par inhalation.

Des exemples non limitatifs de formulations adaptées pour l'inhalation incluent les aérosols (liquides ou solides mis en suspension dans un gaz vecteur) et les sprays (liquides ou solides) à visée nasale.

Selon un mode de réalisation, la deuxième partie dudit nécessaire de pièces (comprenant au moins un agent immunostimulant tel que décrit ci-dessus) est à administrer (ou est pour administration) par voie entérale, par voie topique ou par voie parentérale (voie injectable).

Selon un mode de réalisation, la deuxième partie dudit nécessaire de pièces est à administrer (ou est pour administration) par voie orale, voie buccale, sublinguale, rectale, pulmonaire, percutanée, nasale ou par une voie locale.

Selon un mode de réalisation, la deuxième partie dudit nécessaire de pièces est à administrer (ou est pour administration) par injection sous-cutanée, intraveineuse (IV), intramusculaire, intra-articulaire, intra-synovial, intracisternale, intrathécale, intrahépatique, intralésionnelle ou intra-crâniale.

Selon un mode de réalisation, la deuxième partie dudit nécessaire de pièces comprend une ou plusieurs souches probiotiques et est à administrer (ou est pour administration) par inhalation, par voie orale (entérale *per os*) ou par voie nasale. Ainsi, selon ce mode de réalisation, la deuxième partie dudit nécessaire de pièces est sous une forme adaptée à une administration par inhalation, par voie orale (entérale *per os*) ou par voie nasale.

La présente invention telle que divulguée mais non revendiquée concerne également une méthode pour traiter ou prévenir à long-terme une infection respiratoire et une réinfection respiratoire chez un sujet, dans laquelle la méthode comprend l'administration d'au moins un agent capable de lier l'agent infectieux respiratoire et d'au moins un agent immunostimulant tels que décrits précédemment, l'au moins un agent capable de lier l'agent infectieux étant administré (ou étant formulé pour administration) par inhalation.

La présente invention telle que divulguée mais non revendiquée concerne également l'utilisation d'au moins un agent capable de lier l'agent infectieux respiratoire et d'au moins un agent immunostimulant tels que décrits précédemment pour la fabrication d'un médicament pour traiter ou prévenir à long-terme une infection respiratoire et une réinfection respiratoire chez un sujet, l'au moins un agent capable de lier l'agent infectieux respiratoire étant administré (ou étant formulé pour administration) par inhalation.

Selon un mode de réalisation, l'au moins un agent immunostimulant comprend ou consiste en une ou plusieurs souches probiotiques, de préférence sélectionnée(s) parmi les espèces *Lactobacillacus rhamnosus, Lactobacillacus salivarius* et/ou des espèces proches en terme génomique.

Selon un mode de réalisation, l'au moins un agent immunostimulant est ou comprend un mélange d'une souche probiotique sélectionnée parmi l'espèce *Lactobacillacus rhamnosus* et d'une souche probiotique sélectionnée parmi l'espèce *Lactobacillacus salivarius.*

Selon un mode de réalisation, l'au moins un agent immunostimulant comprend ou consiste en une ou plusieurs souches probiotiques, de préférence sélectionnée(s) parmi l'espèce *Lactobacillacus murinus* et/ou une espèce proche en terme génomique.

Selon un mode de réalisation, l'au moins un agent immunostimulant est sélectionné parmi les souches déposées à la CNCM le 14 avril 2015 sous les numéros CNCM I-4967 et CNCM I-4968, et leur mélange. Selon un mode de réalisation, l'au moins un agent immunostimulant est la souche déposée à la CNCM le 16 Avril 2018 sous le numéro CNCM I-5314.

Selon un mode de réalisation particulier, l'agent immunostimulant est ou comprend un mélange des 2 souches probiotiques déposées à la CNCM le 14 avril 2015 sous les numéros CNCM I-4967 et CNCM I-4968. Selon un mode de réalisation particulier, l'au moins un agent immunostimulant est ou comprend un mélange de la souche probiotique déposée à la CNCM le 14 Avril 2015 sous le numéro CNCM I-4967 et de la souche probiotique déposée à la CNCM le 16 Avril 2018 sous le numéro CNCM I-5314. Selon un mode de réalisation, l'au moins un agent immunostimulant est ou comprend un mélange de la souche probiotique déposée à la CNCM le 14 Avril 2015 sous le numéro CNCM I-4968 et de la souche probiotique déposée à la CNCM le 16 Avril 2018 sous le numéro CNCM I-5314.

Selon un mode de réalisation particulier, l'agent immunostimulant est ou comprend un mélange des 3 souches probiotiques déposées à la CNCM le 14 avril 2015 sous les numéros CNCM I-4967 et CNCM I-4968 et le 16 Avril 2018 sous le numéro CNCM I-5314.

Selon un mode de réalisation, l'agent infectieux respiratoire est un virus, préférentiellement un virus influenza, un VRS, SARS-CoV ou SARS-CoV-2.

Selon un mode de réalisation, l'agent infectieux respiratoire est une bactérie, préférentiellement une bactérie résistante à un ou plusieurs antibiotiques (de préférence du groupe ESKAPE), de préférence une bactérie sélectionnée parmi le groupe comprenant *Pseudomonas aeruginosa, Klebsiella pneumoniae, Staphylococcus aureus* et *Acinetobacter baumanii.,* plus préférentiellement *Pseudomonas aeruginosa.*

Selon un mode de réalisation, l'au moins un agent capable de lier l'agent infectieux respiratoire est dirigé contre une molécule présente à la surface de l'agent infectieux respiratoire, préférentiellement une molécule exprimée à la surface de *Pseudomonas aeruginosa.* Selon un mode de réalisation, l'au moins un agent capable de lier l'agent infectieux respiratoire de la présente invention est dirigé contre une protéine du système de sécrétion, préférentiellement une protéine du système de sécrétion de type III telle que, par exemple, pcrV.

Selon un mode de réalisation, l'au moins un agent capable de lier l'agent infectieux respiratoire est une molécule sélectionnée parmi le groupe comprenant un anticorps, un dérivé d'anticorps tel qu'un fragment d'anticorps, un anticorps multispécifique, un anticorps bispécifique, un anticorps à domaine unique, un unibody ou un nanobody et un mimétique d'anticorps.

Selon un mode de réalisation, l'au moins un agent capable de lier l'agent infectieux respiratoire est un anticorps chimérique, humanisé ou d'origine humaine ou un dérivé de celui-ci.

Selon un mode de réalisation, l'au moins un anticorps ou dérivé d'anticorps utilisé dans la méthode de la présente invention est l'anticorps mAb166 ou un anticorps chimérique ou humanisé issu de l'anticorps mAb166 ou un dérivé de celui-ci.

Selon un mode de réalisation, l'infection respiratoire est une infection respiratoire aigüe, préférentiellement une infection respiratoire des voies inférieures sélectionnée parmi bronchite, la bronchiolite, la pneumonie (par exemple une pneumopathie nosocomiale, une pneumopathie communautaire ou une pneumopathie acquise sous ventilation mécanique), la grippe et la coqueluche.

Selon un mode de réalisation, le sujet souffre d'une pathologie respiratoire chronique, préférentiellement une pathologie respiratoire chronique sélectionnée parmi le groupe comprenant la bronchopneumopathie chronique obstructive (BPCO), les maladies interstitielles pulmonaires, le cancer du poumon, l'asthme (de l'adulte et de l'enfant), la bronchiectasie, les maladies rares et orphelines du poumon comme la mucoviscidose et les maladies vasculaires pulmonaires.

Selon un mode de réalisation, la méthode de la présente invention prévient à court terme l'apparition d'une infection respiratoire chez un sujet.

Selon un mode de réalisation, la méthode de la présente invention induit un effet vaccinal (i.e. protection ou prévention à long-terme) prévenant l'apparition ou la réapparition d'une infection respiratoire chez le sujet.

Selon un mode de réalisation, la méthode de la présente invention comprend l'administration d'une dose d'environ 0.5 à 50 mg de l'au moins un agent capable de lier l'agent infectieux respiratoire, préférentiellement un anticorps ou un dérivé d'anticorps, par kilo de poids, préférentiellement une dose d'environ 1 à 20 mg par kilo, plus préférentiellement une dose d'environ 2.5 à 5 mg par kilo.

Selon un mode de réalisation, la méthode de la présente invention comprend l'administration d'une souche probiotique telle que décrite précédemment à une quantité comprise entre 10³ et 10⁹ ufc (unité formant colonie).

Selon un mode de réalisation, la méthode de la présente invention comprend l'administration d'un mélange de 2 souches probiotiques tel que décrit précédemment à un ratio de 1:1, 1:2, 1:3, 1:4, 1:5, 1:6 ; 1:7, 1:8, 1:9 ou 1:10.

Selon un mode de réalisation, la méthode de la présente invention comprend l'administration d'un mélange de souches probiotiques telles que décrites précédemment où chaque souche est administrée à une quantité comprise entre 10³ et 10⁹ ufc (unité formant colonie).

Selon un mode de réalisation, la méthode de la présente invention comprend l'administration de l'au moins un agent capable de lier l'agent infectieux respiratoire, préférentiellement un anticorps ou un dérivé d'anticorps, et de l'au moins un agent immunostimulant de manière simultanée.

Selon un autre mode de réalisation, la méthode de la présente invention comprend l'administration de l'au moins un agent capable de lier l'agent infectieux respiratoire, préférentiellement un anticorps ou un dérivé d'anticorps, et de l'au moins un agent immunostimulant de manière séparée dans le temps.

Selon un mode de réalisation, la méthode de la présente invention comprend l'administration de l'au moins un agent capable de lier l'agent infectieux respiratoire, préférentiellement un anticorps ou un dérivé d'anticorps, en une seule dose.

Selon un mode de réalisation, la méthode de la présente invention comprend l'administration de l'au moins un agent capable de lier l'agent infectieux respiratoire, préférentiellement un anticorps ou un dérivé d'anticorps, en doses répétées, par exemple une fois, deux fois ou trois fois.

Selon un mode de réalisation, la méthode de la présente invention comprend l'administration d'une souche probiotique ou d'un mélange de souches probiotiques en une seule dose.

Selon un mode de réalisation, la méthode de la présente invention comprend l'administration d'une souche probiotique ou d'un mélange de souches probiotiques en doses répétées, par exemple une fois, deux fois ou trois fois.

Selon un mode de réalisation, la méthode de la présente invention comprend l'administration d'une souche probiotique ou d'un mélange de souches probiotiques pendant au moins 1, 2, 3, 4, 5, 6, 7, 8, 9, 10, 11, 12, 13 ou 14 jours (préférentiellement des jours consécutifs).

Selon un mode de réalisation, l'au moins un agent capable de lier l'agent infectieux respiratoire, préférentiellement un anticorps ou un dérivé d'anticorps, et l'au moins un agent immunostimulant, préférentiellement une souche probiotique ou un mélange de souches probiotiques, sont administrés par des voies d'administration identiques, i.e. par inhalation.

Selon un autre mode de réalisation, l'au moins un agent capable de lier l'agent infectieux respiratoire, préférentiellement un anticorps ou un dérivé d'anticorps, et l'au moins un agent immunostimulant, préférentiellement une souche probiotique ou un mélange de souches probiotiques, sont administrés par des voies d'administration différentes.

Selon un mode de réalisation, une souche probiotique ou un mélange de souches probiotiques de la présente invention sont administrés par inhalation, par voie orale (voie entérale *per os*) ou par voie nasale.

### BRÈVE DESCRIPTION DES DESSINS

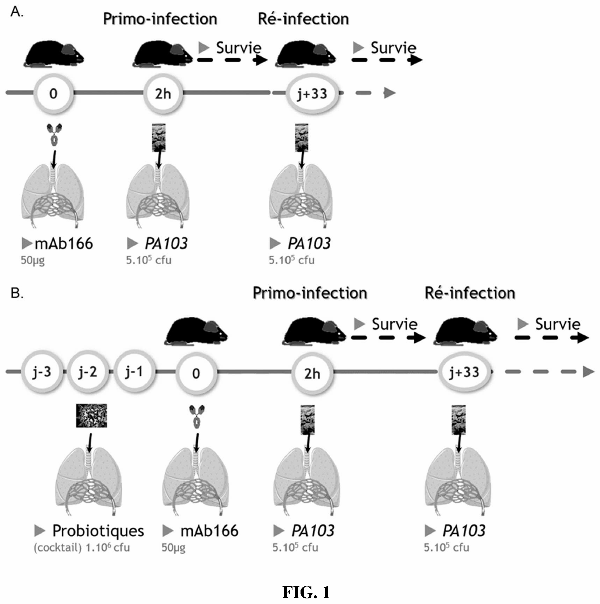

La **Figure 1** est une combinaison de schémas montrant le protocole expérimental du traitement prophylactique par anticorps et/ou anticorps et probiotiques. **Figure 1A** : Des souris C57BL6/jRj (mâles, 7 semaines), reçoivent 50µg de mAb166 via une administration intratrachéale (Microsprayer^{®}). 2 heures plus tard, elles sont infectées avec la souche PA103 de *Pseudomonas aeruginosa,* à la dose de 5x10⁵ufc (unité formant colonie)/40µL/souris via une administration intratrachéale. Un suivi clinique (survie, perte de poids, signes cliniques) des animaux est réalisé pendant 1 mois après cette primo-infection. A J+33, les individus survivants sont réinfectés, sans traitement additionnel. Le suivi clinique est réalisé sur 7 jours. **Figure 1B** : Un cocktail des souches CNCM-I-4967 et CNCM-I-4968, à 1x10⁶ ufc pour chaque souche, est administré aux animaux à J (jour)-3, J-2, J-1 par voie intranasale (40µL/souris), avant le traitement par anticorps et l'infection comme décrits en A.

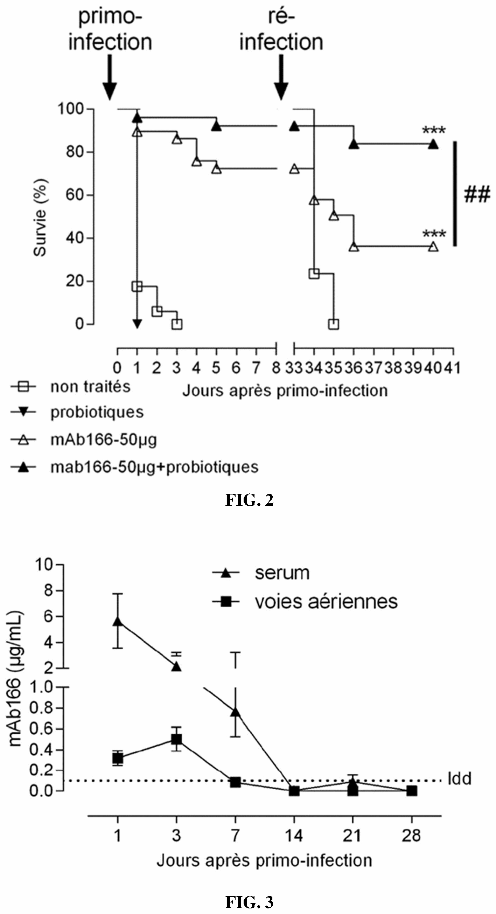

La **Figure 2** est un graphique montrant la survie des individus à la primo-infection et à la réinfection par PA103 après traitement prophylactique par mAb166. La survie des individus traités, selon le protocole décrit en **Figure 1****,** avec mAb166 à 50µg ou un cocktail de probiotiques, ou mAb166 à 50µg combiné à un cocktail de probiotiques ou non traités, a été analysée après la primo-infection et la réinfection. Les résultats sont représentatifs d'un pool de 5 expériences indépendantes (n=10-30). (groupes mAb166-50µg+probiotiques ou mAb166-50µg vs non traités ***: p<0.001 / groupes mAb166-50µg+probiotiques vs mAb166-50µg ## : p<0.01, avec un test de log-rank test).
La **Figure 3** est un graphique montrant l'analyse pharmacocinétique du mAb166 après administration prophylactique. Le dosage du mAb166 total dans le sérum et dans le lavage broncho-alvéolaire (représentatif des voies aériennes inférieures) a été réalisé par test ELISA à 1, 3, 7, 14, 21 et 28 jours après administration de l'anticorps et infection par PA103. Les données sont exprimées en moyenne ± SEM (erreur type de la moyenne). Les résultats sont représentatifs d'un pool de 3-5 expériences indépendantes (n=5-30 / point d'analyse). La ligne en pointillée indique la limite de détection (ldd) du dosage.

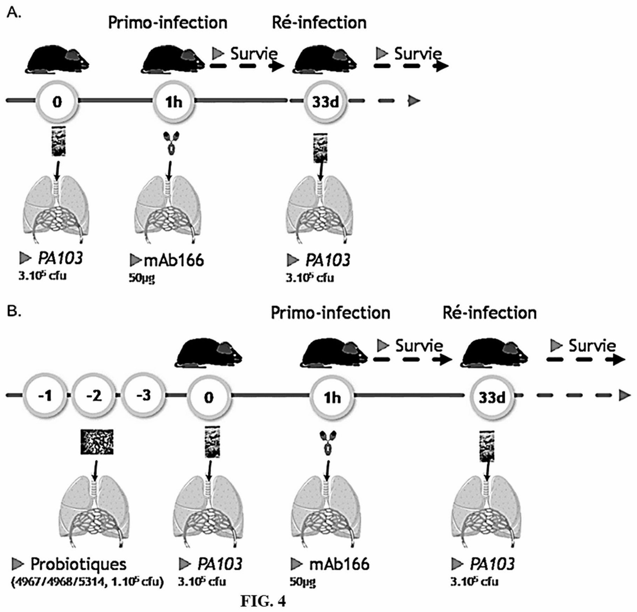

La **Figure 4** est une combinaison de schémas montrant le protocole expérimental du traitement thérapeutique par anticorps et/ou anticorps et probiotiques. **Figure 4A** : Des souris C57BL6/jRj (mâles, 7 semaines) sont infectées avec la souche PA103 de *Pseudomonas aeruginosa,* à la dose de 3x10⁵ufc/40µL/souris via une administration intratrachéale puis reçoivent 1 heure plus tard 50µg de mAb166 via une administration intratrachéale (Microsprayer^{®}). Un suivi clinique (survie, perte de poids, signes cliniques) des animaux est réalisé pendant 1 mois après cette primo-infection. A J+33, les individus survivants sont réinfectés, sans traitement additionnel. Le suivi clinique est réalisé sur 7 jours. **Figure 4B** : Les souches CNCM-I-4967, CNCM-I-4968, ou CNCM-I-5314 à 1x10⁵ ufc pour chaque souche, sont administrées aux animaux à J-3, J-2, J-1 par voie intranasale (40µL/souris), avant l'infection et le traitement par anticorps comme décrits en A.

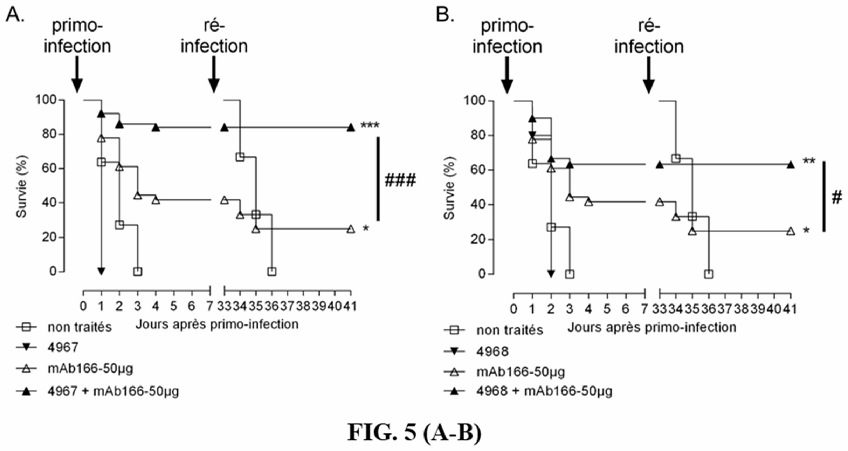

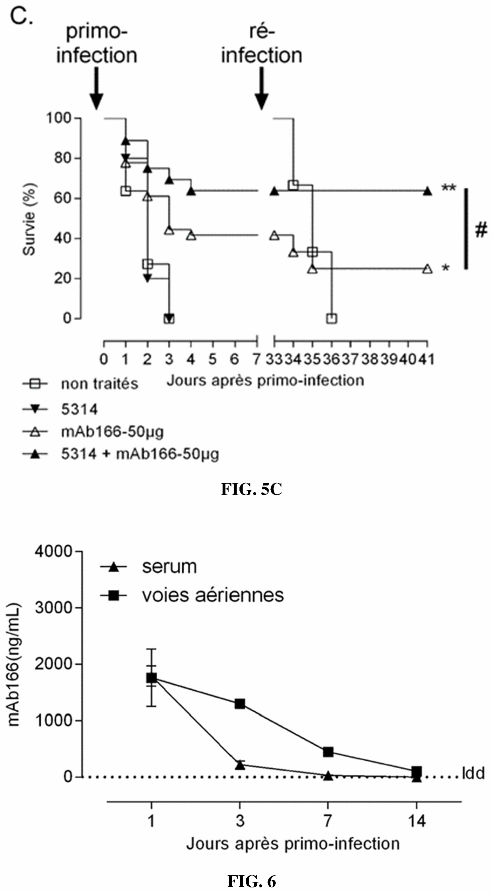

La **Figure 5** est une combinaison de graphiques montrant la survie des individus à la primo-infection et à la réinfection par PA103 après traitement thérapeutique par mAb166. **Figure 5A** : Souche CNCM-I-4967 : Les résultats sont représentatifs d'un pool de 3 expériences indépendantes (n=5-50). (groupes mAb166-50µg+4967 ou mAb166-50µg vs non traités * : p<0.05 ; ***: p<0.001 / groupes mAb166-50µg+4967 vs mAb166-50µg ### : p<0.001, avec un test de log-rank test). **Figure 5B** : Souche CNCM-I-4968 : Les résultats sont représentatifs d'un pool de 3 expériences indépendantes (n=5-30). (groupes mAb166-50µg+4967 ou mAb166-50µg vs non traités * : p<0.05 ; ***: p<0.001 / groupes mAb166-50µg+4967 vs mAb166-50µg ### : p<0.001, avec un test de log-rank test). **Figure 5C** **:** Souche CNCM-I-5314 : Les résultats sont représentatifs d'un pool de 3 expériences indépendantes (n=5-35). (groupes mAb166-50µg+4967 ou mAb166-50µg vs non traités * : p<0.05 ; ***: p<0.001 / groupes mAb166-50µg+4967 vs mAb166-50µg ### : p<0.001, avec un test de log-rank test).
La **Figure 6** est un graphique montrant l'analyse pharmacocinétique du mAb166 après administration thérapeutique. Le dosage du mAb166 total dans le sérum et dans le lavage broncho-alvéolaire (représentatif des voies aériennes inférieures) a été réalisé par test ELISA à 1, 3, 7, 14, 21 et 28 jours après administration de l'anticorps et infection par PA103. Les données sont exprimées en moyenne ± SEM. Les résultats sont représentatifs d'un pool de 3-5 expériences indépendantes (n=5-30 / point d'analyse). La ligne en pointillée indique la limite de détection (ldd) du dosage.

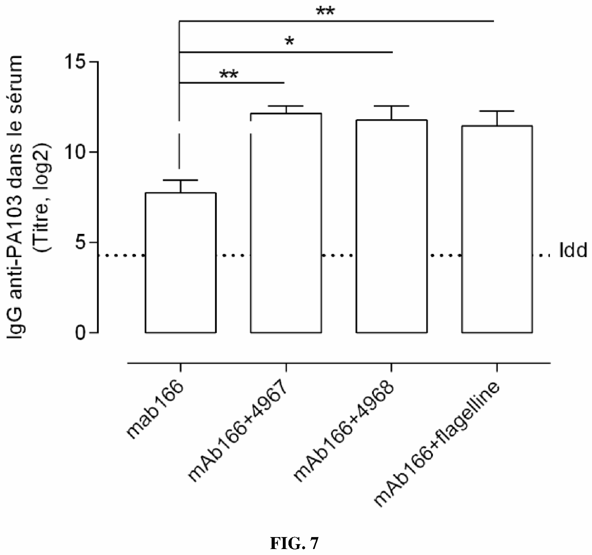

La **Figure 7** est un graphique montrant la réponse humorale anti-PA103 dans le sérum à la réinfection par PA103 après traitement thérapeutique par mAb166. La quantité d'immunoglobuline G spécifique de PA103 dans les individus traités, selon le protocole décrit en **Figure 4B****,** avec mAb166 à 50µg, ou mAb166 à 50µg combiné à une souche probiotique ou mAb166 à 50µg combiné à la Flagelline (FLAMOD, 2.5µg/souris), a été analysée après la réinfection par un test ELISA semi-quantitatif. Les résultats sont représentatifs d'un pool de 2 expériences indépendantes (n=5-10). (groupes mab166 vs combo *: p<0.05 ; **: p<0.01, avec un test Anova). La ligne en pointillée indique la limite de détection (ldd) du dosage.

### EXEMPLES

La présente invention se comprendra mieux à la lecture de l'exemple suivant qui illustre non-limitativement l'invention.

### Example 1 : Traitement prophylactique par anticorps et/ou anticorps et probiotiques

### Matériel et Méthodes

### Protocole expérimental

Dans ces expériences, la souche de *Pseudomonas aeruginosa* PA103, a été utilisée comme représentative des souches impliquées dans des infections respiratoires aigües.

Des souris C57/BL6jRj (mâles, 7 semaines) ont été infectées une première fois par PA103 avec un dépôt pulmonaire de l'inoculum bactérien (primo-infection). Les bactéries ont été diluées dans du PBS, pour obtenir un titre de 5.10⁵ bactéries/40 µL.

Deux heures au préalable, les souris avaient reçu une administration par inhalation de l'anticorps mAb166 (50 µg), une IgG2b contrôle (clone MPC11), ou d'une solution de PBS via une administration intratrachéale en utilisant un MicroSprayer^{®} aerosolizer (Penn-Century, USA) **(****Figure 1A****).** Le mAb166 est une IgG2b murine spécifique de la protéine pcrV, un composant essentiel du système de sécrétion de type 3 exprimé à la surface de PA103.

Pour le traitement des souris avec des souches probiotiques, les souches CNCM I-4967 et CNCM I-4968, issues de poumons de souris naïves et déposées le 14 avril 2015 à la CNCM, ont été utilisées. Ces souches ont été initialement identifiées comme appartenant aux espèces *Lactobacillus rhamnosus* et *Lactobacillus salivarius,* respectivement ou à des espèces proches en terme génomique. Des analyses de séquences ont montré que ces souches appartiennent à l'espèce *Lactobacillus murinus.*

Les souris ont reçu un mélange des souches probiotiques CNCM I-4967 et 4968 (10⁶ ufc de chaque souche, 40µL par souris) par inhalation par voie intranasale 1, 2 et 3 jours avant le priming **(****Figure 1B****).**

Après l'infection, la survie et l'évolution de leur poids sont suivis quotidiennement. Afin d'interroger une protection à long-terme induite par l'anticorps mAb166, les animaux survivant à la primo-infection, sont réinfectés (infection secondaire) à J+33 après la primo-infection, sans traitement additionnel. Leur survie et la réponse immunitaire associées sont analysées.

### Analyse pharmacocinétique du mAb166 après administration prophylactique.

Le dosage du mAb166 total dans le sérum et dans le lavage broncho-alvéolaire (représentatif des voies aériennes inférieures) a été réalisé par test ELISA à 1, 3, 7, 14, 21 et 28 jours après administration de l'anticorps et infection par PA103.

### Résultats

### Protection à court-terme induite par un anticorps inhalé contre Pa (Pseudomonas aeruginosa) en combinaison avec des souches probiotiques

Comme indiqué dans la **Figure 2****,** les données révèlent que les animaux traités avec l'anticorps anti-Pa mAb166 (50 µg) administré par inhalation montrent une survie très significativement améliorée en comparaison des animaux non traités (*i.e.* ayant reçu la solution de PBS) lors d'une primo-infection (première infection). Cependant, cette protection n'est que partielle, puisque le taux de survie atteint environ 70%. De plus, des données révèlent que l'administration d'une IgG2b contrôle (clone MPC11) n'a aucun impact positif sur la survie des animaux traités lors d'une primo-infection (données non montrées).

Ces données révèlent également que les animaux traités avec une combinaison de l'anticorps inhalé anti-Pa mAb166 (50 µg) avec un mélange des souches probiotiques CNCM I-4967 et CNCM-4968 montrent une survie supérieure (plus de 90%) par rapport aux animaux non traités ou traités avec l'anticorps anti-Pa mAb166 seul lors d'une première infection **(****Figure 2****).** En revanche, l'administration seule des souches probiotiques CNCM I-4967 et CNCM-4968 n'a pas d'incidence sur la survie.

Ainsi, les données indiquent que la combinaison d'un anticorps anti-Pa inhalé avec un mélange de souches probiotiques est plus efficace pour traiter une première infection respiratoire à Pa.

### Protection à long-terme induite par un anticorps inhalé contre Pa en combinaison avec des souches probiotiques

Les animaux survivants ont été réinfectés à J+33 après la primo-infection, alors que l'anticorps anti-Pa mAb166 n'était plus détectable dans le sang et les voies aériennes **(****Figure 3****),** avec la même dose de Pa que lors de la primo-infection.

Les animaux ayant été traités avec l'anticorps anti-Pa mAb166 seul présentent une meilleure survie (moins de 40%) après la réinfection (ou seconde infection) par rapport aux animaux n'ayant pas été traités **(****Figure 2****),** montrant ainsi que les animaux infectés par Pa et traités avec un anticorps inhalé présentent une réponse mémoire leur permettant de contrôler une réinfection (ou seconde infection) (par le même pathogène) et ceci en l'absence de l'administration d'un traitement additionnel lors de la réinfection.

De façon intéressante, les animaux ayant été traités avec la combinaison de l'anticorps anti-Pa mAb166 et le mélange de souches probiotiques montrent une survie nettement supérieure (autour de 80%) à celle des animaux n'ayant pas été traités ou ayant été traités avec l'anticorps anti-Pa mAb166 seul **(****Figure 2****).** L'administration des souches probiotiques seules n'a quant à elle aucun effet significatif. Ces données révèlent donc que la combinaison d'un anticorps inhalé avec le mélange de souches probiotiques est plus efficace que l'anticorps seul pour contrôler une réinfection (ou seconde infection) par le même pathogène.

Ainsi, ces données démontrent que l'administration d'une combinaison d'un anticorps anti-infectieux inhalé avec un mélange de souches probiotiques au moment de la primo-infection permet d'améliorer l'efficacité à court-terme et à long terme d'un anticorps anti-infectieux.

### Exemple 2 : Traitement thérapeutique par anticorps et/ou anticorps et probiotiques

### Matériel et Méthodes

### Protocole expérimental

Des souris C57BL6/jRj (mâles, 7 semaines) ont été infectées avec la souche PA103 de *Pseudomonas aeruginosa,* à la dose de 3x10⁵ ufc/40µL/souris par inhalation, via une administration intratrachéale, puis ont reçu 1 heure plus tard 50µg de mAb166 via une administration intratrachéale (Microsprayer^{®}) **(****Figure 4A****).** Un suivi clinique (survie, perte de poids, signes cliniques) des animaux a été réalisé pendant 1 mois après cette primo-infection. A J+33, les individus survivants ont été réinfectés, sans traitement additionnel. Le suivi clinique a été réalisé sur 7 jours.

Pour le traitement des souris avec des souches probiotiques, les souches CNCM-I-4967, CNCM-I-4968, ou CNCM-I-5314 à 1x10⁵ ufc pour chaque souche, ont été administrées aux animaux à J-3, J-2, J-1 par inhalation par voie intranasale (40µL/souris), avant l'infection et le traitement par anticorps comme décrits dans la **Figure 4B****.**

### Analyse pharmacocinétique du mAb166 après administration thérapeutique

Le dosage du mAb166 total dans le sérum et dans le lavage broncho-alvéolaire (représentatif des voies aériennes inférieures) a été réalisé par test ELISA à 1, 3, 7, 14, 21 et 28 jours après l'infection par PA103 et administration de l'anticorps.

### Résultats

### Traitement à court-terme induit par un anticorps inhalé contre Pa (Pseudomonas aeruginosa) en combinaison avec des souches probiotiques

Les résultats en Figure **5A-C** montrent que les animaux traités avec l'anticorps en combinaison avec l'une des souches CNCM I-4967, CNCM I-4968 ou CNCM I-5314 ont une meilleure survie en comparaison des animaux non traités ou traités par l'anticorps mAb166 seul lors d'une première infection. En revanche, l'administration seule de l'une des souches probiotiques n'a pas d'incidence positive sur la survie.

Ces données montrent que la combinaison d'un anticorps anti-Pa inhalé avec une souche probiotique est plus efficace pour traiter une première infection à Pa.

### Traitement à long-terme induit par un anticorps inhalé contre Pa en combinaison avec des souches probiotiques

Les animaux survivants ont été réinfectés à J+33 après la primo-infection, alors que l'anticorps mAb166 n'était plus détectable dans le sang et les voies aériennes **(****Figure 6****),** avec la même dose de Pa que lors de la primo-infection.

Les animaux ayant été traités avec l'anticorps mAb166 seul présentent une meilleure survie (environ 30%) après la réinfection (ou seconde infection) par rapport aux animaux n'ayant pas été traités **(****Figure 5A-C****),** montrant ainsi que les animaux infectés par Pa et traités avec un anticorps inhalé présentent une réponse mémoire leur permettant de contrôler une seconde infection (par le même pathogène).

En revanche, les animaux ayant été traités avec la combinaison de l'anticorps mAb166 et l'une des souches CNCM I-4967, CNCM I-4968 ou CNCM I-5314 montrent une survie plus élevée (entre 60 et 80%) que celle des animaux n'ayant pas été traités ou ayant été traités avec l'anticorps mAb166 seul **(****Figure 5A-C****).**

Ainsi, ces données révèlent que l'administration lors de la primo-infection d'une combination d'un anticorps anti-Pa inhalé avec une souche probiotique permet de contrôler à la fois une première infection respiratoire mais aussi des infections respiratoires répétées, alors qu'il n'y a plus d'anticorps anti-Pa ou de souches probiotiques inhalés dans l'organisme.

### Exemple 3 : Réponse humorale induite lors de la réinfection par P. aeruginosa suite au traitement lors de la primo-infection par un anticorps anti-Pa, un anticorps anti-Pa combiné à une souche probiotique et un anticorps anti-Pa combiné à la flagelline

### Matériels et Méthodes

La quantité d'immunoglobuline G spécifique de PA103 dans les individus traités, selon le protocole décrit en **Figure 4B****,** avec mAb166 à 50µg, ou mAb166 à 50µg combinée à une souche probiotique ou mAb166 à 50µg combinée à de la Flagelline (FLAMOD, 2.5µg/souris), a été analysée 5 jours après la réinfection par un test ELISA semi-quantitatif.

### Résultats

Les résultats de la **Figure 7** indiquent que lors de la réinfection, la combinaison d'un anticorps anti-Pa mAb166 inhalé avec une souche probiotique telle que CNCM I-4967 et CNCM I-4968 induit une plus grande quantité d'immunoglobuline G spécifique de PA103 dans le sérum des animaux traités que le traitement par un anticorps anti-Pa mAb166 seul.

De façon intéressante, lors de la réinfection, la combinaison d'un anticorps anti-Pa mAb166 inhalé en combinaison avec la flagelline induit également une plus grande quantité d'immunoglobuline G spécifique de PA103 dans le sérum des animaux traités que le traitement par un anticorps anti-Pa mAb166 seul **(****Figure 7****).** Il est à noter que les individus traités avec la flagelline seule lors de la primo-infection ne résistent pas à l'infection.

Ces résultats démontrent que la réponse humorale lors de la réinfection est meilleure lorsqu'un anticorps anti-infectieux inhalé a été utilisé en combinaison avec une souche probiotique ou la flagelline que le traitement par un anticorps seul au moment de la primo-infection, permettant ainsi de prévenir efficacement les infections respiratoires répétées sur le long-terme.

## Revendications

1. Une combinaison d'au moins un agent capable de lier un agent infectieux respiratoire, ledit agent étant choisi parmi un anticorps, un anticorps bispécifique, un anticorps multispécifique, un fragment d'anticorps, un anticorps à domaine unique, un unibody, un nanobody, un affibody, une affiline, une affitine, une adnectine, une atrimère, une DARPin, une anticaline, une avimère, une fynomère ou un versabody, et d'au moins un agent immunostimulant, pour son utilisation dans le traitement ou la prévention à long-terme d'une infection respiratoire et d'une réinfection respiratoire chez un sujet, dans laquelle l'au moins un agent capable de lier l'agent infectieux respiratoire est à administrer par inhalation chez le sujet, étant entendu par long-terme que le traitement ou la prévention perdure alors que l'au moins un agent capable de lier l'agent infectieux n'est plus détecté dans l'organisme et/ou lorsqu'une réponse immunitaire adaptative est induite dans l'organisme.

2. La combinaison d'au moins un agent capable de lier un agent infectieux respiratoire et d'au moins un agent immunostimulant pour son utilisation selon la revendication **1,** dans laquelle l'au moins un agent immunostimulant est sélectionné parmi le groupe comprenant ou consistant en une souche probiotique, un mélange de souches probiotiques, un agoniste des récepteurs de type Toll, un agoniste des récepteurs de type NOD, un agonistes des récepteurs de type RIG, une cytokine ou un mélange de cytokines, une chémokine ou un mélange de chémokines, un adjuvant tel que le chitosan, une flagelline, un variant de flagelline dans lequel un ou plusieurs résidu(s) d'acide aminé donné a/ont été modifié(s) sans altérer la conformation globale et la fonction de la flagelline, un polypeptide comprenant ou consistant en un ou plusieurs fragment(s) de flagelline, un oligodésoxynucléotide CpG, l'α-galactosylcéramide, les sels d'aluminium tels que l'hydroxyde d'aluminium, le phosphate d'aluminium, et le sulfate de potassium et d'aluminium, le MF59, le AS03, l'acide polyinosinique25 polycytidylique, un polyphosphazène, un anticorps dirigé contre les points du contrôle du système immunitaire tels que CTLA-4, PD-1, PD-Ll ou CD137 et leurs mélanges.

3. La combinaison d'au moins un agent capable de lier un agent infectieux respiratoire et d'au moins un agent immunostimulant pour son utilisation selon l'une quelconque des revendications **1** et **2,** dans laquelle l'au moins un agent immunostimulant est ou comprend une souche probiotique ou un mélange de souches probiotiques sélectionnée(s) parmi la famille *Lactobacillaceae,* préférentiellement parmi l'espèce *Lactobacillus murinus.*

4. La combinaison d'au moins un agent capable de lier un agent infectieux respiratoire et d'au moins un agent immunostimulant pour son utilisation selon l'une quelconque des revendications **1** à **3,** dans laquelle l'au moins un agent immunostimulant est ou comprend une souche probiotique sélectionnée parmi les souches déposées à la Collection Nationale de Cultures de Microorganismes (CNCM) le 14 avril 2015 sous les numéros CNCM 1-4967 et CNCM 1-4968, ou la souche déposée le 16 Avril 2018 sous le numéro CNCM 1-5314, ou un mélange de celles-ci.

5. La combinaison d'au moins un agent capable de lier un agent infectieux respiratoire et d'au moins un agent immunostimulant pour son utilisation selon l'une quelconque des revendications **1** à **4,** dans laquelle l'agent infectieux respiratoire est sélectionné parmi le groupe comprenant ou consistant en les virus, bactéries, champignons et parasites, préférentiellement dans laquelle l'agent infectieux respiratoire est au moins temporairement extracellulaire.

6. La combinaison d'au moins un agent capable de lier un agent infectieux respiratoire et d'au moins un agent immunostimulant pour son utilisation selon l'une quelconque des revendications **1** à **5,** dans laquelle l'agent infectieux respiratoire est une bactérie, préférentiellement une bactérie résistante à un ou plusieurs antibiotiques, plus préférentiellement une bactérie sélectionnée parmi le groupe ESKAPE, encore plus préférentiellement une bactérie sélectionnée dans le groupe comprenant ou consistant en *Pseudomonas aeruginosa, Klebsiella pneumoniae, Staphylococcus aureus* et *Acinetobacter baumanii,* encore plus préférentiellement *Pseudomonas aeruginosa.*

7. La combinaison d'au moins un agent capable de lier un agent infectieux respiratoire et d'au moins un agent immunostimulant pour son utilisation selon l'une quelconque des revendications **1** à **6,** dans laquelle l'au moins l'au moins un agent capable de lier l'agent infectieux respiratoire est dirigé contre une molécule présente à la surface d'une bactérie.

8. La combinaison d'au moins un agent capable de lier un agent infectieux respiratoire et d'au moins un agent immunostimulant pour son utilisation selon la revendication **7,** dans laquelle l'au moins un agent capable de lier l'agent infectieux respiratoire est dirigé contre une molécule présente à la surface de *Pseudomonas aeruginosa,* préférentiellement l'au moins un agent capable de lier l'agent infectieux respiratoire est dirigé contre une protéine du système de sécrétion de type III de *Pseudomonas aeruginosa.*

9. La combinaison d'au moins un agent capable de lier un agent infectieux respiratoire et d'au moins un agent immunostimulant pour son utilisation selon l'une quelconque des revendications **1** à **8,** dans laquelle ladite infection respiratoire est une infection respiratoire aigüe, préférentiellement une infection respiratoire aigüe des voies inférieures, plus préférentiellement la bronchite, la bronchiolite, la pneumonie, la pneumopathie nosocomiale, la pneumopathie communautaire, la pneumopathie acquise sous ventilation mécanique, la grippe ou la coqueluche.

10. La combinaison d'au moins un agent capable de lier un agent infectieux respiratoire et d'au moins un agent immunostimulant pour son utilisation selon l'une quelconque des revendications **1** à **9,** dans laquelle le sujet souffre d'une pathologie respiratoire chronique, préférentiellement une pathologie respiratoire chronique sélectionnée parmi le groupe comprenant ou consistant en la bronchopneumopathie chronique obstructive (BPCO), les maladies interstitielles pulmonaires, le cancer du poumon, l'asthme de l'adulte et de l'enfant, la bronchiectasie, les maladies rares et orphelines du poumon comme la mucoviscidose et les maladies vasculaires pulmonaires.

11. Une composition comprenant ou consistant essentiellement en une combinaison d'au moins un agent capable de lier un agent infectieux respiratoire et d'au moins un agent immunostimulant pour son utilisation selon l'une quelconque des revendications **l** à **10.**

12. Un nécessaire de pièces comprenant au moins deux parties, la première partie comprenant au moins un agent capable de lier un agent infectieux respiratoire, ledit agent étant sélectionné parmi un anticorps, un anticorps bispécifique, un anticorps multispécifique, un fragment d'anticorps, un anticorps à domaine unique, un unibody, un nanobody, un affibody, une affiline, une affitine, une adnectine, une atrimère, une DARPin, une anticaline, une avimère, une fynomère et un versabody, et la seconde partie comprenant au moins un agent immunostimulant pour son utilisation dans le traitement ou la prévention à long-terme d'une infection respiratoire et d'une réinfection respiratoire chez un sujet, dans lequel l'au moins un agent capable de lier l'agent infectieux respiratoire est à administrer par inhalation chez le sujet, étant entendu par long-terme que le traitement ou la prévention perdure alors que l'au moins un agent capable de lier l'agent infectieux n'est plus détecté dans l'organisme et/ou lorsqu'une réponse immunitaire adaptative est induite dans l'organisme.

13. Nécessaire de pièces selon la revendication **12,** dans lequel l'au moins un agent immunostimulant est sélectionné parmi le groupe comprenant ou consistant en une souche probiotique, un mélange de souches probiotiques, un agoniste des récepteurs de type Toll, un agoniste des récepteurs de type NOD, un agonistes des récepteurs de type RIG, une cytokine ou un mélange de cytokines, une chémokine ou un mélange de chémokines, un adjuvant tel que le chitosan, une flagelline, un variant de flagelline dans lequel un ou plusieurs résidu(s) d'acide aminé donné a/ont été modifié(s) sans altérer la conformation globale et la fonction de la flagelline, un polypeptide comprenant ou consistant en un ou plusieurs fragment(s) de flagelline, un oligodésoxynucléotide CpG, l'α-galactosylcéramide, les sels d'aluminium tels que l'hydroxyde d'aluminium, le phosphate d'aluminium, et le sulfate de potassium et d'aluminium, le MF59, le AS03, l'acide polyinosinique-polycytidylique, un polyphosphazène, un anticorps dirigé contre les points du contrôle du système immunitaire tels que CTLA-4, PD-1, PD-Ll ou CD137 et leurs mélanges.

## Patentansprüche

1. Kombination aus mindestens einem Mittel, das in der Lage ist, ein respiratorisches infektiöses Agens zu binden, wobei das Mittel ausgewählt ist aus einem Antikörper, einem bispezifischen Antikörper, einem multispezifischen Antikörper, einem Antikörperfragment, einem Einzeldomänenantikörper, einem Unibody, einem Nanobody, einem Affibody, einem Affilin, einem Affitin, einem Adnectin, einem Atrimer, einem DARPin, einem Anticalin, einem Avimer, einem Fynomer oder einem Versabody, und mindestens einem immunstimulierenden Mittel zur Verwendung bei der langfristigen Behandlung oder Prävention einer respiratorischen Infektion und einer respiratorischen Reinfektion bei einem Individuum, wobei das mindestens eine Mittel, das in der Lage ist, das respiratorische infektiöse Agens zu binden, durch Inhalation an das Individuum zu verabreichen ist, wobei sich unter langfristig versteht, dass die Behandlung oder Prävention fortgesetzt wird, nachdem das mindestens eine Mittel, das in der Lage ist, das infektiöse Agens zu binden, nicht mehr im Körper nachgewiesen wird und/oder wenn eine adaptive Immunantwort im Körper induziert wird.

2. Kombination aus mindestens einem Mittel, das in der Lage ist, ein respiratorisches infektiöses Agens zu binden, und mindestens einem immunstimulierenden Mittel zur Verwendung nach Anspruch 1, wobei das mindestens eine immunstimulierende Mittel ausgewählt ist aus der Gruppe umfassend oder bestehend aus einem probiotischen Stamm, einer Mischung von probiotischen Stämmen, einem Agonisten von Toll-like-Rezeptoren, einem Agonisten von NOD-like-Rezeptoren, einem Agonisten von RIG-like-Rezeptoren, einem Zytokin oder Gemisch von Zytokinen, einem Chemokin oder Gemisch von Chemokinen, einem Adjuvans wie Chitosan, einem Flagellin, einer Flagellinvariante, bei der ein oder mehrere gegebene Aminosäurerest(e) modifiziert wurde(n), ohne die Gesamtkonformation und Funktion des Flagellins zu verändern, einem Polypeptid, das ein oder mehrere Flagellinfragment(e) umfasst oder daraus besteht, einem CpG-Oligodesoxynukleotid, α-Galactosylceramid, Aluminiumsalzen, wie Aluminiumhydroxid, Aluminiumphosphat und Kaliumaluminiumsulfat, MF59, AS03, Polyinosin25 - Polycytidylsäure, einem Polyphosphazen, einem Antikörper, der gegen Immunsystem-Checkpoints wie CTLA-4, PD-1, PD-L1 oder CD137 gerichtet ist, und Mischungen davon.

3. Kombination aus mindestens einem Mittel, das in der Lage ist, ein respiratorisches infektiöses Agens zu binden, und mindestens einem immunstimulierenden Mittel zur Verwendung nach einem der Ansprüche 1 und 2, wobei das mindestens eine immunstimulierende Mittel ein probiotischer Stamm oder eine Mischung von probiotischen Stämmen ist oder umfasst, welche(r) aus der Familie *Lactobacillaceae*, vorzugsweise aus der Spezies *Lactobacillus murinus*, ausgewählt ist (sind).

4. Kombination aus mindestens einem Mittel, das in der Lage ist, ein respiratorisches infektiöses Agens zu binden, und mindestens einem immunstimulierenden Mittel zur Verwendung nach einem der Ansprüche 1 bis 3, wobei das mindestens eine immunstimulierende Mittel ein probiotischer Stamm ist oder diesen umfasst, ausgewählt aus den Stämmen, die am 14. April 2015 unter den Nummern CNCM 1-4967 und CNCM 1-4968 bei der Collection Nationale de Cultures de Microorganismes (CNCM) hinterlegt wurden, oder dem Stamm, der am 16. April 2018 unter der Nummer CNCM 1-5314 hinterlegt wurde, oder einer Mischung davon.

5. Kombination aus mindestens einem Mittel, das in der Lage ist, ein respiratorisches infektiöses Agens zu binden, und mindestens einem immunstimulierenden Mittel zur Verwendung nach einem der Ansprüche 1 bis 4, wobei das respiratorische infektiöse Agens aus der Gruppe ausgewählt ist, die Viren, Bakterien, Pilzen und Parasiten umfasst oder daraus besteht, wobei vorzugsweise das respiratorische infektiöse Agens zumindest zeitweise extrazellulär vorliegt.

6. Kombination aus mindestens einem Mittel, das in der Lage ist, ein respiratorisches infektiöses Agens zu binden, und mindestens einem immunstimulierenden Mittel zur Verwendung nach einem der Ansprüche 1 bis 5, wobei das respiratorische infektiöse Agens ein Bakterium ist, vorzugsweise ein Bakterium, das gegen ein oder mehrere Antibiotika resistent ist, stärker bevorzugt ein Bakterium ausgewählt aus der Gruppe ESKAPE, noch bevorzugter ein Bakterium ausgewählt aus der Gruppe, umfassend oder bestehend aus *Pseudomonas aeruginosa, Klebsiella pneumoniae, Staphylococcus aureus* und *Acinetobacter baumanii,* noch weiter bevorzugt *Pseudomonas aeruginosa.*

7. Kombination aus mindestens einem Mittel, das in der Lage ist, ein respiratorisches infektiöses Agens zu binden, und mindestens einem immunstimulierenden Mittel zur Verwendung nach einem der Ansprüche 1 bis 6, wobei das mindestens eine Mittel, das in der Lage ist, das respiratorische infektiöse Agens zu binden, gegen ein Molekül gerichtet ist, das auf der Oberfläche eines Bakteriums vorhanden ist.

8. Kombination aus mindestens einem Mittel, das in der Lage ist, ein respiratorisches infektiöses Agens zu binden, und mindestens einem immunstimulierenden Mittel zur Verwendung nach Anspruch 7, wobei das mindestens eine Mittel, das in der Lage ist, das respiratorische infektiöse Agens zu binden, gegen ein Molekül gerichtet ist, das auf der Oberfläche von *Pseudomonas aeruginosa* vorhanden ist, wobei vorzugsweise das mindestens eine Mittel, das in der Lage ist, das respiratorische infektiöse Agens zu binden, gegen ein Protein des Typ-III-Sekretionssystems von *Pseudomonas aeruginosa* gerichtet ist.

9. Kombination aus mindestens einem Mittel, das in der Lage ist, ein respiratorisches infektiöses Agens zu binden, und mindestens einem immunstimulierenden Mittel zur Verwendung nach einem der Ansprüche 1 bis 8, wobei es sich bei der respiratorischen Infektion um eine akute respiratorische Infektion, vorzugsweise um eine akute respiratorische Infektion der unteren Atemwege, besonders bevorzugt um Bronchitis, Bronchiolitis, Pneumonie, nosokomiale Pneumonitis, gemeinschaftlich erworbene Pneumonitis, Pneumonitis, die unter mechanischer Beatmung erworben wurde, Influenza oder Keuchhusten handelt.

10. Kombination aus mindestens einem Mittel, das in der Lage ist, ein respiratorisches infektiöses Agens zu binden, und mindestens einem immunstimulierenden Mittel zur Verwendung nach einem der Ansprüche 1 bis 9, wobei das Individuum an einer chronischen respiratorischen Pathologie leidet, vorzugsweise einer chronischen Atemwegspathologie, ausgewählt aus der Gruppe umfassend, oder bestehend aus, chronische obstruktive Lungenerkrankung (COPD), interstitielle Lungenerkrankungen, Lungenkrebs, Asthma bei Erwachsenen und Kindern, Bronchiektasie, seltene bzw. Orphan-Krankheiten der Lunge, wie zystische Fibrose, und pulmonale Gefäßerkrankungen.

11. Zusammensetzung, umfassend oder im Wesentlichen bestehend aus einer Kombination aus mindestens einem Mittel, das in der Lage ist, ein respiratorisches infektiöses Agens zu binden, und mindestens einem immunstimulierenden Mittel, zur Verwendung nach einem der Ansprüche 1 bis 10.

12. Kit von Teilen, umfassend mindestens zwei Teile, wobei der erste Teil mindestens ein Mittel umfasst, das in der Lage ist, ein respiratorisches infektiöses Agens zu binden, wobei das Mittel ausgewählt ist aus einem Antikörper, einem bispezifischen Antikörper, einem multispezifischen Antikörper, einem Antikörperfragment, einem Einzeldomänenantikörper, einem Unibody, einem Nanobody, einem Affibody, einem Affilin, einem Affitin, einem Adnectin, einem Atrimer, einem DARPin, einem Anticalin, einem Avimer, einem Fynomer oder einem Versabody, und wobei der zweite Teil mindestens ein immunstimulierendes Mittel umfasst, zur Verwendung bei der langfristigen Behandlung oder Prävention einer respiratorischen Infektion und einer respiratorischen Reinfektion bei einem Individuum, wobei das mindestens eine Mittel, das in der Lage ist, das respiratorische infektiöse Agens zu binden, durch Inhalation an das Individuum zu verabreichen ist, wobei sich unter langfristig versteht, dass die Behandlung oder Prävention fortgesetzt wird, nachdem das mindestens eine Mittel, das in der Lage ist, das infektiöse Agens zu binden, nicht mehr im Körper nachgewiesen wird und/oder wenn eine adaptive Immunantwort im Körper induziert wird.

13. Kit von Teilen nach Anspruch 12, wobei das mindestens eine immunstimulierende Mittel ausgewählt ist aus der Gruppe umfassend oder bestehend aus einem probiotischen Stamm, einer Mischung von probiotischen Stämmen, einem Agonisten von Toll-like-Rezeptoren, einem Agonisten von NOD-like-Rezeptoren, einem Agonisten von RIG-like-Rezeptoren, einem Zytokin oder Gemisch von Zytokinen, Chemokin oder Gemisch von Chemokinen, einem Adjuvans wie Chitosan, einem Flagellin, einer Flagellinvariante, bei der ein oder mehrere gegebene Aminosäurerest(e) modifiziert wurde(n), ohne die Gesamtkonformation und Funktion des Flagellins zu verändern, einem Polypeptid, das ein oder mehrere Flagellinfragment(e) umfasst oder daraus besteht, einem CpG-Oligodesoxynukleotid, α-Galactosylceramid, Aluminiumsalzen, wie Aluminiumhydroxid, Aluminiumphosphat und Kaliumaluminiumsulfat, MF59, AS03, Polyinosin-Polycytidylsäure, einem Polyphosphazen, einem Antikörper, der gegen Immunsystem-Checkpoints wie CTLA-4, PD-1, PD-L1 oder CD137 gerichtet ist, und Mischungen davon.

## Claims

1. A combination of at least one agent capable of binding a respiratory infectious agent, said agent being selected from an antibody, a bispecific antibody, a multispecific antibody, an antibody fragment, a single-domain antibody, a unibody, a nanobody, an affibody, an affilin, an affitin, an adnectin, an atrimer, a DARPin, an anticalin, an avimer, a fynomer or a versabody, and of at least one immunostimulatory agent, for use thereof in the long-term treatment or prevention of a respiratory infection and respiratory reinfection in a subject, wherein the at least one agent capable of binding the respiratory infectious agent is to be administered to the subject by inhalation, "long-term" being understood as meaning that the treatment or the prevention persists even though the at least one agent capable of binding the infectious agent is no longer detected in the body and/or when an adaptive immune response is induced in the body.

2. The combination of at least one agent capable of binding a respiratory infectious agent and of at least one immunostimulatory agent for use thereof according to Claim 1, wherein the at least one immunostimulatory agent is selected from the group comprising or consisting of a probiotic strain, a mixture of probiotic strains, a Toll-like receptor agonist, an NOD-like receptor agonist, a RIG-like receptor agonist, a cytokine or a mixture of cytokines, a chemokine or a mixture of chemokines, an adjuvant such as chitosan, a flagellin, a flagellin variant in which one or more given amino acid residues have been modified without altering the overall conformation and the function of the flagellin, a polypeptide comprising or consisting of one or more flagellin fragments, a CpG oligodeoxynucleotide, α-galactosylceramide, aluminium salts such as aluminium hydroxide, aluminium phosphate, and potassium aluminium sulfate, MF59, AS03, polyinosinic-polycytidylic acid, a polyphosphazene, an antibody directed against immune system checkpoints such as CTLA-4, PD-1, PD-L1 or CD137, and mixtures thereof.

3. The combination of at least one agent capable of binding a respiratory infectious agent and of at least one immunostimulatory agent for use thereof according to either one of Claims 1 and 2, wherein the at least one immunostimulatory agent is or comprises a probiotic strain or a mixture of probiotic strains selected from the family *Lactobacillaceae*, preferentially from the species *Lactobacillus murinus*.

4. The combination of at least one agent capable of binding a respiratory infectious agent and of at least one immunostimulatory agent for use thereof according to any one of Claims 1 to 3, wherein the at least one immunostimulatory agent is or comprises a probiotic strain selected from strains deposited with the Collection Nationale de Cultures de Microorganismes [French National Collection of Cultures of Microorganisms] (CNCM) on 14 April 2015 under the numbers CNCM 1-4967 and CNCM 1-4968, or the strain deposited on 16 April 2018 under the number CNCM 1-5314, or a mixture thereof.

5. The combination of at least one agent capable of binding a respiratory infectious agent and of at least one immunostimulatory agent for use thereof according to any one of Claims 1 to 4, wherein the respiratory infectious agent is selected from the group comprising or consisting of viruses, bacteria, fungi and parasites, preferentially wherein the respiratory infectious agent is at least temporarily extracellular.

6. The combination of at least one agent capable of binding a respiratory infectious agent and of at least one immunostimulatory agent for use thereof according to any one of Claims 1 to 5, wherein the respiratory infectious agent is a bacterium, preferentially a bacterium resistant to one or more antibiotics, more preferentially a bacterium selected from the ESKAPE group, more preferentially still a bacterium selected from the group comprising or consisting of *Pseudomonas aeruginosa*, *Klebsiella pneumoniae*, *Staphylococcus aureus* and *Acinetobacter baumanii*, more preferentially still *Pseudomonas aeruginosa*.

7. The combination of at least one agent capable of binding a respiratory infectious agent and of at least one immunostimulatory agent for use thereof according to any one of Claims 1 to 6, wherein the at least one agent capable of binding the respiratory infectious agent is directed against a molecule present on the surface of a bacterium.

8. The combination of at least one agent capable of binding a respiratory infectious agent and of at least one immunostimulatory agent for use thereof according to Claim 7, wherein the at least one agent capable of binding the respiratory infectious agent is directed against a molecule present on the surface of *Pseudomonas aeruginosa*; preferentially, the at least one agent capable of binding the respiratory infectious agent is directed against a protein of the type III secretion system of *Pseudomonas aeruginosa*.

9. The combination of at least one agent capable of binding a respiratory infectious agent and of at least one immunostimulatory agent for use thereof according to any one of Claims 1 to 8, wherein said respiratory infection is an acute respiratory infection, preferentially an acute lower respiratory tract infection, more preferentially bronchitis, bronchiolitis, pneumonia, nosocomial pneumonia, community-acquired pneumonia, ventilator-associated pneumonia, influenza or whooping cough.

10. The combination of at least one agent capable of binding a respiratory infectious agent and of at least one immunostimulatory agent for use thereof according to any one of Claims 1 to 9, wherein the subject is suffering from a chronic respiratory pathology, preferentially a chronic respiratory pathology selected from the group comprising or consisting of chronic obstructive pulmonary disease (COPD), pulmonary interstitial diseases, lung cancer, adult and pediatric asthma, bronchiectasis, rare and orphan lung diseases such as cystic fibrosis and pulmonary vascular diseases.

11. A composition comprising or consisting essentially of a combination of at least one agent capable of binding a respiratory infectious agent and of at least one immunostimulatory agent for use thereof according to any one of Claims 1 to 10.

12. A kit of components comprising at least two parts, the first part comprising at least one agent capable of binding a respiratory infectious agent, said agent being selected from an antibody, a bispecific antibody, a multispecific antibody, an antibody fragment, a single-domain antibody, a unibody, a nanobody, an affibody, an affilin, an affitin, an adnectin, an atrimer, a DARPin, an anticalin, an avimer, a fynomer and a versabody, and the second part comprising at least one immunostimulatory agent, for use thereof in the long-term treatment or prevention of a respiratory infection and respiratory reinfection in a subject, wherein the at least one agent capable of binding the respiratory infectious agent is to be administered to the subject by inhalation, "long-term" being understood as meaning that the treatment or the prevention persists even though the at least one agent capable of binding the infectious agent is no longer detected in the body and/or when an adaptive immune response is induced in the body.

13. Kit of components according to Claim 12, wherein the at least one immunostimulatory agent is selected from the group comprising or consisting of a probiotic strain, a mixture of probiotic strains, a Toll-like receptor agonist, an NOD-like receptor agonist, a RIG-like receptor agonist, a cytokine or a mixture of cytokines, a chemokine or a mixture of chemokines, an adjuvant such as chitosan, a flagellin, a flagellin variant in which one or more given amino acid residues have been modified without altering the overall conformation and the function of the flagellin, a polypeptide comprising or consisting of one or more flagellin fragments, a CpG oligodeoxynucleotide, α-galactosylceramide, aluminium salts such as aluminium hydroxide, aluminium phosphate, and potassium aluminium sulfate, MF59, AS03, polyinosinic-polycytidylic acid, a polyphosphazene, an antibody directed against immune system checkpoints such as CTLA-4, PD-1, PD-L1 or CD137, and mixtures thereof.
